# EUROPEAN PATENT SPECIFICATION

(11) **EP 1 957 528 B1**
(45) Date of publication and mention of the grant of the patent: **07.11.2012**
(21) Application number: 06818135.3
(22) Date of filing: 30.11.2006
(51) Int. Cl.: C07K 14/705

(54) **A NUCLEOTIDE VACCINE**
NUCLEOTID-VAKZINE
VACCIN NUCLÉOTIDIQUE

(30) Priority: 30.11.2005 DK 200501697; 30.12.2005 DK 200501857; 30.12.2005 US 755712 P; 27.03.2006 DK 200600431
(43) Date of publication of application: 20.08.2008
(73) Proprietor: University of Copenhagen, 1017 Copenhagen K (DK)
(72) Inventor: HOLST, Peter Johannes, DK-2700 Bronshoj (DK); THOMSEN, Allan Randrup, DK-2100 Copenhagen Ø (DK); CHRISTENSEN, Jan Pravsgaard, DK-2650 Hvidovre (DK)
(74) Representative: Zwicker, Jörk
(86) International application number: PCT/DK2006/000675
(87) International publication number: WO 2007/062656

(56) References cited:
- WO-A-97/10335
- DE-A1- 10 228 218
- MALCHEREK G ET AL: "MHC CLASS II-ASSOCIATED INVARIANT CHAIN PEPTIDE REPLACEMENT BY T CELL EPITOPES: ENGINEERED INVARIANT CHAIN AS A VEHICLE FOR DIRECTED AND ENHANCED MHC CLASS II ANTIGEN PROCESSING AND PRESENTATION" EUROPEAN JOURNAL OF IMMUNOLOGY, WEINHEIM, DE, vol. 28, no. 5, May 1998 (1998-05), pages 1524-1533, XP001206094 ISSN: 0014-2980
- VAN TIENHOVEN E A E ET AL: "Induction of antigen specific CD4+ T cell responses by invariant chain based DNA vaccines" VACCINE, BUTTERWORTH SCIENTIFIC. GUILDFORD, GB, vol. 19, no. 11-12, 8 December 2001 (2001-12-08), pages 1515-1519, XP004313967 ISSN: 0264-410X
- DE MARCO FEDERICO ET AL: "DNA vaccines against HPV-16 E7-expressing tumour cells." ANTICANCER RESEARCH, vol. 23, no. 2B, March 2003 (2003-03), pages 1449-1454, XP009084130 ISSN: 0250-7005
- NAGATA T ET AL: "Induction of a single helper T-cell epitope-specific T cells by Th-oriented minigene DNA vaccine against Listeria monocytogenes." ABSTRACTS OF THE GENERAL MEETING OF THE AMERICAN SOCIETY FOR MICROBIOLOGY, vol. 103, 2003, pages E-022 URL, XP009084146 & 103RD AMERICAN SOCIETY FOR MICROBIOLOGY GENERAL MEETING; WASHINGTON, DC, USA; MAY 18-22, 2003 ISSN: 1060-2011
- HOLST P J ET AL: "'Molecular modification of antigens delivered by replication deficient adenovirus greatly improves immunogenicity'" IMMUNOLOGY, vol. 120, no. Suppl. 1, March 2007 (2007-03), page 13, XP009084090 & ANNUAL CONGRESS OF THE BRITISH-SOCIETY-OF-IMMUNOLOGY; GLASGOW, UK; FEBRUARY 20 -23, 2007 ISSN: 0019-2805
- DATABASE Geneseq [Online] 29 January 2003 (2003-01-29), "Human expressed protein tag (EPT) #1935." XP002434464 retrieved from EBI accession no. GSP:ABU05269 Database accession no. ABU05269
- HUMBERT MARTINE ET AL: "The invariant chain induces compact forms of class II molecules localized in late endosomal compartments" EUROPEAN JOURNAL OF IMMUNOLOGY, vol. 23, no. 12, 1993, pages 3158-3166, XP002434456 ISSN: 0014-2980
- ANTOINE J-C ET AL: "LOCALIZATION OF MAJOR HISTOCOMPATIBILITY COMPLEX CLASS II MOLECULES IN PHAGOLYSOSOMES OF MURINE MACROPHAGES INFECTED WITH LEISHMANIA-AMAZONENSIS" INFECTION AND IMMUNITY, vol. 59, no. 3, 1991, pages 764-775, XP002434457 ISSN: 0019-9567

## Description

### Field of the invention

The present invention relates to a technology and method whereby a faster, broader and stronger immune response is obtained when using viral and DNA-based vaccines.

### Background of the invention

Despite current knowledge in the field of immunology especially regarding vaccine technologies no suitable vaccines are available against numerous pathogens. Widespread pandemics of HIV (Human Immunodeficiency Virus), HTLV (Human T-cell Lymphotropic Virus), tuberculosis and HCV (Hepatitis C virus) remain out of reach of effective vaccination, while bird flu and other emerging pathogens threaten to overwhelm our healthcare systems. Similarly, the burst in world wide terrorism has expanded the potential epidemics to include exotic and lethal pathogens such as Ebola, Lassa and Marburg.

Vaccines can be prophylactic: they are given before the actual infection occurs, or therapeutic: where they elicit or accelerate an immune response to a pathogen already in the body. Both methods of vaccination require the establishment of a solid immune response. The immune response that is activated by infection or vaccination depends on the interaction of several cell types, such as T-, B- and antigen presenting cells as well as several different molecules, primarily antigens, MHC molecules, T- and B-cells receptors and many more.

Antigens are peptide fragments presented on the surface of antigen presenting cells by MHC molecules. Antigens can be of foreign, i.e. pathogenic origin, or stem from the organism itself, so called self or auto antigens. The MHC molecules are representatives of a polymorphous gene family encoded by a specific chromosomal region known as the "major histocompatibility complex", hence MHC. Two classes of MHC molecules exist, MHC class I (MHC-I) and MHC class II (MHC-II).

T-helper cells are stimulated by antigens presented by MHC class II (MHC-II) molecules residing on the surface of antigen presenting cells. The MHC-II molecules are synthesized in the endoplasmatic reticulum. During synthesis, they combine with invariant chain (li) in a manner preventing the MHC-II molecules from being loaded with self- or auto-antigens. The MHC-II molecule is by signal sequences in the invariant chain transported to the cell surface in a specific cellular compartment. As the compartment matures by the processing of its contents it progresses from being a lysosome, to a late endosome (after fusion with endocytotic vesicles) to an MHC class II compartment (MIIC). The endocytotic vesicle contains foreign antigen i.e. proteolytically cleaved bacterial peptide fragments. These fragments are by their degradation prepared to be loaded onto the MHC-II molecule. The MHC-II molecule is released by the invariant chain in a two part process when the invariant chain first is degraded proteolytically leaving only a peptide termed CLIP in the MHC-II binding domain, secondly by the removal of CLIP by an HLA-DM molecule Stumptner et al EMBO 1997, 16, 5807-5818 defines the CLIP region. The MHC-II molecule is then free to bind the foreign antigens and present these on the cell surface after fusion of the MIIC vesicle to the plasma membrane. This initiates the humoral immune response as the presented antigen stimulates activation of a T-helper cell which in turn by several means activates a B cell, which ultimately differentiates into an antibody secreting cell.

The cellular immune response is initiated when the T-cell receptor of T-cytotoxic cells recognizes antigen bound to the MHC class I molecule on an antigen presenting cell. MHC-I molecules are not associated with a molecule of a functionality like the invariant chain that associates with MHC-II. The processing of MHC-I into an antigen presenting molecule furthermore differs from that of MHC-II molecules in that the MHC-I molecule is loaded with antigen already in the endoplasmatic reticulum. The antigens presented by the MHC-I molecule are typically peptide fragments cleaved by the proteasome of proteins that have been synthesized by the antigen presenting cell itself. These proteins may be abnormal proteins encoded in the cells own DNA or proteins derived from viruses or other pathogens that have infected the cell and parasitize its protein synthesis machinery. The MHC class I-related proteolytic system is present in virtually all cells.

The functions of the two types of T cells are significantly different, as implied by their names. Cytotoxic T cells eradicate intracellular pathogens and tumors by direct lysis of cells and by secreting cytokines such as γ-interferon. The predominant cytotoxic T cell is the CD8⁺ T cell, which also is antigen specific. Helper T cells also can lyse cells, but their primary function is to secrete cytokines that promote the activities of B cells (antibody-producing cells) and other T cells and thus they broadly enhance the immune response to foreign antigens, including antibody-mediated and cytotoxic T cell-mediated response mechanisms. CD4⁺ T cells are the major helper T cell phenotype in the immune response.

Traditional vaccines rely on whole organisms, either pathogenic strains that have been killed or strains with attenuated pathogenicity. On the one hand, these vaccines run the risk of introducing the disease they are designed to prevent if the attenuation is insufficient or if enough organisms survive the killing step during vaccine preparation. On the other hand, such vaccines have reduced infectivity and are often insufficiently immunogenic, resulting in inadequate protection from the vaccination.

Recently, molecular biological techniques have been used in an attempt to develop new vaccines based on individual antigenic proteins from the pathogenic organisms. Conceptually, use of antigenic peptides rather than whole organisms would avoid pathogenicity while providing a vaccine containing the most immunogenic antigens. However, it has proven difficult to select the optimal antigen of a given protein or polypeptide and furthermore it has been found that pure peptides or carbohydrates tend to be weak immunogens.

Genetic (DNA) vaccines are new and promising candidates for the development of both prophylactic and therapeutic vaccines. The strength of the ensuing immune response is determined through a combination of the potency of the vector (i.e. naked DNA, viral vectors, live attenuated viruses etc.), the expression level of the antigen, and the recombinant antigen it self (i.e. high or low affinity MHC binders, structural determinants selecting for more or less limited T-or B-cell repertoire etc.). It is generally held to be true, that efficient induction of immunological memory requires or benefits from the interactions of CD4⁺ (helper cell) T-cells with CD8⁺ (cytotoxic) T-cells and B-cells that mediate many of the effects of immune memory. However, one potential disadvantage of conventional DNA vaccines is their low immunogenicity in humans. One likely cause of this low immunogenicity is the restricted access of antigens formed within cells to the MHC II pathway for antigen processing and presentation to T helper cells.

### Summary of invention

Thus, the present invention has solved the problem of stimulating the immune response in a manner that increases the kinetics of the response, simultaneously with both broadening and improving the response, while avoiding, among other things, the above mentioned disadvantages of the vaccination methods described in the state of the art. In particular, a novel system for a directed, specific and fast stimulation of the immune system is hereby made available in order to improve the vaccination of all animals.

This problem is solved by the embodiments of the present invention characterized in the claims. By the present invention it was found that fusion of an antigen to the invariant chain dramatically enhanced the ensuing antiviral CD4⁺ and CD8⁺ T-cell responses. Additionally, and surprisingly, it was found that this effect is obtained through a CD4⁺ T-cell independent mechanism. It was further found that the protection is both accelerated and enhanced in an acute localized and lethal infection, and enhanced in a high-dose systemic infection.

It is thus an object of the present invention to provide a nucleic acid construct comprising sequences encoding at least one invariant chain operatively linked to at least one antigenic protein or peptide or an antigenic fragment of said protein or peptide.

It is likewise an aspect of the present invention to provide an adenoviral vector comprising a nucleotide construct encoding at least one antigen and at least one protein or peptide or fragment of a protein or peptide which stimulates an immune response.

Thus it is an aspect of the present invention to provide means of stimulating an MHC-I mediated immune response by an adenoviral vector comprising a nucleotide construct encoding at least one antigen and at least one protein or peptide or fragment of a protein or peptide.

Another aspect includes stimulating an MHC-II response by an adenoviral vector comprising a nucleotide construct encoding at least one antigen and at least one protein or peptide or fragment of a protein or peptide.

A further aspect provides means of stimulating intercellular spreading of the nucleic acid construct, the adenoviral vector, the proteins encoded within any of these or any parts of any of these.

It is further an object of the present invention to provide a delivery vehicle comprising the nucleic acid construct as detailed herein, especially a delivery vehicle such as a replication deficient adenoviral vector is relevant to the present invention.

It is yet an object and an aspect of the present invention to provide a cell comprising the nucleic acid construct or the adenoviral vector according to the present invention.

It is yet an object of the present invention to provide a chimeric protein as encoded by the nucleic acid construct described herein or encoded by the adenoviral vector described herein.

It is further an aspect of the present invention to provide an antibody that recognizes the chimeric protein encoded by either the nucleic acid construct or the adenoviral vector described herein.

It is an aspect of the present invention to provide a vaccine comprising the nucleic acid construct or the adenoviral vector as detailed herein. Especially relevant to the present invention is a vaccine, where at least one invariant chain is operatively linked to at least one protein or peptide or fragment of a protein or peptide which stimulates an MHC-I response. This may be done by operatively linking at least one invariant chain with at least one at least one antigenic protein or peptide or an antigenic fragment of said protein or peptide.

It is further an aspect of the present invention to provide a vaccine comprising the chimeric protein encoded within the nucleic acid construct or the adenoviral vector as detailed herein.

It is yet an aspect of the present invention to provide a kit in parts, said kit comprising either a vaccine composition comprising an adenoviral vector or a nucleic acid construct as described herein together with a medical instrument or other means of administering said vaccine and furthermore instructions on how to use the kit in parts.

It follows that the present invention provides means for inducing an immune response in an animal, by administering to the animal a vaccine comprising the nucleic acid construct or the adenoviral vector as detailed herein below.

### Description of Drawings

- **Figure 1:**: Schematic drawing of inserts in the adenovirus vector.
- **Figure 2:**: CD8⁺ and CD4⁺ T-cell responses to adenovirus encoded epitopes.
- **Figure 3:**: CD8⁺ and CD4⁺ T-cell responses to adenovirus encoded epitopes in F₁ hybrid mice.
- **Figure 4:**: Ad-liGP exerts CD8⁺ T-cell stimulatory effects that are independent of CD4⁺ T-cells
- **Figure 5:**: Ad-liGP confers rapid and superior protection against lethal LCMV infection.
- **Figure 6:**: Ad-liGP efficiently protects against high-dose, intravenous LCMV infection.
- **Figure 7:**: Ad-liGP confers superior protection to lethal LCMV variants with mutations in immunodominant epitopes.
- **Figure 8:**: Frequencies of CD8⁺ or CD4⁺T cells reacting to specific LCMV epitopes after Ad-liGP vaccination and challenge with LCMV variants with mutations in immunodominant epitopes.
- **Figure 9:**: CD8⁺ and CD4⁺ T cell responses to vaccination with naked DNA-liGP and DNA-GP
- **Figure 10:**: Prophylactic vaccination with Ad-li-GP increases tumor rejection
- **Figure 11:**: Therapeutic vaccination with Ad-li-GP increases average life span in tumor carrying mice.
- **Figure 12:**: Survival rate following vaccination with either Ad-li-VSVGP or Ad-VSVGP.
- **Figure 13:**: CD8⁺ and CD4⁺ T-cell responses to more adenovirus encoded epitopes
- **Figure 14:**: Efficiency of Ad-li-GP constructs compared to Ad-GP-Lamp-1 constructs measured by CD8⁺ T-cell responses to adenovirus encoded epitopes.
- **Figure 15:**: Vector based on in-frame polylinkers.
- **Figure 16:**: Vectors with IRES2 sites

### Detailed description of the invention

### Definitions:

**Adenovirus:** A group of double-stranded DNA containing viruses. Adenoviruses can be genetically modified making them replication incompetent or conditionally replication incompetent. In this form, as adenoviral constructs or adenovectors, they can be used as gene delivery vehicles for vaccination or gene therapy.

**Adjuvant:** Any substance whose admixture with an administered immunogenic determinant / antigen / nucleic acid construct increases or otherwise modifies the immune response to said determinant.

**Amino acid:** Any synthetic or naturally occurring amino carboxylic acid, including any amino acid occurring in peptides and polypeptides including proteins and enzymes synthesized *in vivo* thus including modifications of the amino acids. The term amino acid is herein used synonymously with the term "amino acid residue" which is meant to encompass amino acids as stated which have been reacted with at least one other species, such as 2, for example 3, such as more than 3 other species. The generic term amino acid comprises both natural and non-natural amino acids any of which may be in the "D" or "L" isomeric form.

**Antibody:** Immunoglobulin molecules and active portions of immunoglobulin molecules. Antibodies are for example intact immunoglobulin molecules or fragments thereof retaining the immunologic activity.

**Antigen:** Any substance that can bind to a clonally distributed immune receptor (T-cell or B-cell receptor). Usually a peptide, polypeptide or a multimeric polypeptide. Antigens are preferably capable of eliciting an immune response.

**Boost:** To boost by a booster shot or dose is to give an additional dose of an immunizing agent, such as a vaccine, given at a time after the initial dose to sustain the immune response elicited by the previous dose of the same agent.

**Carrier:** Entity or compound to which antigens are coupled to aid in the induction of an immune response.

**Chimeric protein:** A genetically engineered protein that is encoded by a nucleotide sequence made by a splicing together of two or more complete or partial genes or a series of (non)random nucleic acids.

**Complement:** A complex series of blood proteins whose action "complements" the work of antibodies. Complement destroys bacteria, produces inflammation, and regulates immune reactions.

**Cytokine:** Growth or differentiation modulator, used non-determinative herein, and should not limit the interpretation of the present invention and claims. In addition to the cytokines, adhesion or accessory molecules, or any combination thereof, may be employed alone or in combination with the cytokines.

**CTL:** Cytotoxic T lymphocytes. A sub group of T-cells expressing CD8 along with the T-cell receptor and therefore able to respond to antigens presented by class I molecules.

**Delivery vehicle:** An entity whereby a nucleotide sequence or polypeptide or both can be transported from at least one media to another.

**Fragment:** is used to indicate a non-full length part of a nucleic acid or polypeptide. Thus, a fragment is itself also a nucleic acid or polypeptide, respectively.

**Individual:** Any species or subspecies of bird, mammal, fish, amphibian, or reptile.

**Invariant chain:** an integral membrane protein glycoprotein that associates with and stabilizes MHC II molecules in the endoplasmatic reticulum and subsequent cellular compartments. Here the term invariant chain covers all naturally occurring or artificially generated full length or fragmented homologous genes and proteins of a certain similarity to human invariant chain. Invariant chain is herein abbreviated li.

**Isolated:** used in connection with nucleic acids, polypeptides, and antibodies disclosed herein 'isolated' refers to these having been identified and separated and/or recovered from a component of their natural, typically cellular, environment. Nucleic acids, polypeptides, and antibodies of the invention are preferably isolated, and vaccines and other compositions of the invention preferably comprise isolated nucleic acids, polypeptides or isolated antibodies.

**MHC:** Major histocompatibility complex, two main subclasses of MHC, Class I and Class II exist.

**Nucleic acid:** A chain or sequence of nucleotides that convey genetic information. In regards to the present invention the nucleic acid is a deoxyribonucleic acid (DNA).

**Nucleic acid construct:** A genetically engineered nucleic acid. Typically comprising several elements such as genes or fragments of same, promoters, enhancers, terminators, polyA tails, linkers, polylinkers, operative linkers, multiple cloning sites (MCS), markers, STOP codons, other regulatory elements, internal ribosomal entry sites (IRES) or others.

**Operative linker:** A sequence of nucleotides or amino acid residues that bind together two parts of a nucleic acid construct or (chimeric) polypeptide in a manner securing the biological processing of the nucleic acid or polypeptide.

**Pathogen:** a specific causative agent of disease, especially a biological agent such as a virus, bacteria, prion or parasite that can cause disease to its host, also referred to as an infective agent.

**Peptide:** Plurality of covalently linked amino acid residues defining a sequence and linked by amide bonds. The term is used analogously with oligopeptide and polypeptide. The natural and/or non-natural amino acids may be linked by peptide bonds or by non-peptide bonds. The term peptide also embraces post-translational modifications introduced by chemical or enzyme-catalyzed reactions, as are known in the art. The term can refer to a variant or fragment of a polypeptide.

**Pharmaceutical carriers:** also termed excipients, or stabilizers are non-toxic to the cell or individual being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN.TM., polyethylene glycol (PEG), and PLURONICS.TM. **Plurality:** At least two.

**Promoter:** A binding site in a DNA chain at which RNA polymerase binds to initiate transcription of messenger RNA by one or more nearby structural genes.

**Signal peptide:** A short sequence of amino acids that determine the eventual location of a protein in the cell, also referred to as sorting peptide.

**siRNA:** Small interfering RNAs (siRNAs), which target (in a sequence-specific manner) endogenous RNAs for degradation, thereby reducing the amount of a gene product.

**Surfactant:** A surface active agent capable of reducing the surface tension of a liquid in which it is dissolved. A surfactant is a compound containing a polar group which is hydrophilic and a non polar group which is hydrophobic and often composed of a fatty chain.

**Vaccine:** A substance or composition capable of inducing an immune response in an animal. Also referred to as an immunogenic composition in the present text. An immune response being an immune response (humoral/antibody and/or cellular) inducing memory in an organism, resulting in the infectious agent, being met by a secondary rather than a primary response, thus reducing its impact on the host organism. A vaccine of the present invention may be given as or prophylactic and/or therapeutic medicament. The composition may comprise one or more of the following: antigen(s), nucleic acid constructs comprising one or more antigens operatively linked to li, carriers, adjuvants and pharmaceutical carriers.

**Variant:** a 'variant' of a given reference nucleic acid or polypeptide refers to a nucleic acid or polypeptide that displays a certain degree of sequence homology/identity to said reference nucleic acid or polypeptide but is not identical to said reference nucleic acid or polypeptide.

The present invention relates to a vaccine comprising a nucleic acid construct such as a DNA construct especially a nucleic acid construct comprising sequences encoding invariant chain operatively linked to antigenic protein or peptide encoding sequences. The vaccine stimulates an immune response, especially an immune response in an MHC-I dependent, but CD4⁺ T-cell independent manner.

### Nucleic acid construct

An aspect of the present invention relates to nucleic acid constructs comprising sequences encoding at least one invariant chain operatively linked to at least one antigenic protein or peptide or an antigenic fragment of said protein or peptide, in short an antigen.

By nucleic acid construct is understood a genetically engineered nucleic acid. The nucleic acid construct may be a non-replicating and linear nucleic acid, a circular expression vector, an autonomously replicating plasmid or viral expression vector. A nucleic acid construct may comprise several elements such as, but not limited to genes or fragments of same, promoters, enhancers, terminators, poly-A tails, linkers, polylinkers, operative linkers, multiple cloning sites (MCS), markers, STOP codons, internal ribosomal entry sites (IRES) and host homologous sequences for integration or other defined elements. Methods for engineering nucleic acid constructs are well known in the art (see, e.g., Molecular Cloning: A Laboratory Manual, Sambrook et al., eds., Cold Spring Harbor Laboratory, 2nd Edition, Cold Spring Harbor, N.Y., 1989).

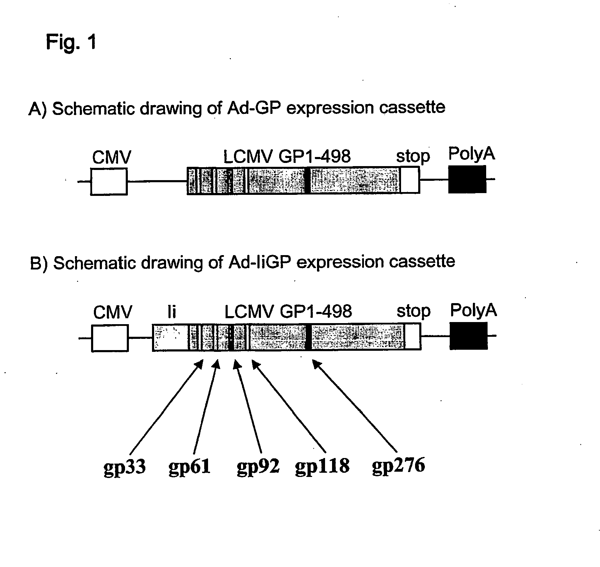

Examples of parts of nucleic acid constructs are given in figures 1, 15 and 16, as well as in the sequences identified as SEQ ID NO: 5, 6, 7, 8, 9, and 10. The partial vector sequences of SEQ ID NO: 5, 6, 7, 8, 9, and 10 have been generated by subcloning various elements as described in the above and as illustrated in figures 1, 15 and 16. These partial sequences are all inserted into the pAC-CMVpLpARS(+) vector (Becker et al., 1994, Methods Cell Biol. 43 Pt A:161-189), see GenBank accession number AY590429.1.

### Invariant chain

The invariant chain (ii) or CD74, is a non-polymorphic type II integral membrane protein, see SEQ ID NOs: 2 and 4 for the amino acid sequences of human and mouse li, respectively, and likewise SEQ ID NOs: 1 and 3 for the nucleic acid sequences of human and mouse li, respectively. Invariant chain has multiple functions in lymphocyte maturation and in adaptive immune responses, in particular targeting to lysosomal compartments were the li CLIP sequence can occupy MHC class II molecules until these are fused with endosomal compartments (Pieters J. 1997, Curr. Opin. Immunol., 9:8996). Additionally li has been shown to function as an MHC class I chaperone (Morris et al, 2004, Immunol. Res. 30:171-179) and by its endosomal targeting sequence, to facilitate stimulation of CD4⁺, but not CD8⁺ T-cells directed against covalently linked antigen (Diebold et al., 2001, Gene Ther. 8:487-493).

The invariant chain protein comprises several domains: a cytosolic domain which includes a signal or sorting peptide (also known as the lysosomal targeting sequence), a transmembrane domain, and a luminal domain which in itself comprises a CLIP region, KEY region, core domain and trimerization domain. Both of these domains are flanked by highly flexible regions (Strumptner-Cuvelette & Benaroch, 2002, Biochem. Biophys. Acta., 1542:1-13). Invariant chain has been characterized in several organisms, including vertebrates (e.g. chicken), mammals (e.g. cow, dog, mouse and rat) and human.

The present invention relates to nucleic acid constructs comprising sequences wherein at least one invariant chain is organism specific or can be related to a specific organism. Preferably, at least one invariant chain is of vertebrate origin, more preferably of mammalian origin and most preferably of human origin. In relation hereto the sequence defined by SEQ ID NO: 1 is the nucleic acid sequence of the invariant chain from human. The employed invariant chain is preferably the invariant chain of the organism that is to receive the vaccination. It is an object of the present invention that the invariant chain and the host organisms or receivers of the treatment are of the same species.

The present invention also relates to a nucleic acid construct wherein the encoded at least one invariant chain is a fragment of the sequence identified in SEQ ID NO: 2 of at least 40 amino acids and of at least 85% identity to the same fragment of SEQ ID NO: 2.

The fragment is a fragment of at least 40 amino acids from any part of the invariant chain as set forth in SEQ ID NO: 2. This includes a fragment including residues 1 to 40,10 to 50, 20 to 60, 25 to 65, 30 to 70, 35 to 75, 40 to 80, 45 to 85, 50 to 90, 55 to 95, 60 to 100, 65 to 105, 70 to 110, 75 to 115, 80 to 120, 85 to 125, 90 to 130, 95 to 135, 100 to 140, 105 to 145, 110 to 150, 115 to 155, 120 to 160, 125 to 165, 130 to 170, 135 to 175, 140 to 180, 145 to 185, 150 to 190, 155 to 195, 160 to 200,165 to 205, 170 to 210 and 175 to 216. It also includes fragments as any of the above listed expanding up to 5 residues to either side hereof. It further includes fragment of at least 50 residues, of at least 60 residues, of at least 70 residues, of at least 80 residues, of at least 90 residues, of at least 100 residues, of at least 110 residues, of at least 120 residues, of at least 130 residues, of at least 140 residues, of at least 150 residues, of at least 160 residues, of at least 170 residues, of at least 180 residues of at least 190 residues, of at least 200 residues and of at least 210 residues.

Any of the above described fragments of at least 85 % sequence identity, for example at least 90 % sequence identity, for example at least 91 % sequence identity, such as at least 92 % sequence identity, for example at least 93 % sequence identity, such as at least 94 % sequence identity, for example at least 95 % sequence identity, such as at least 96 % sequence identity, for example at least 97% sequence identity, such as at least 98 % sequence identity, for example 99% sequence identity with SEQ ID NO: 2 are included within the scope of the present invention.

The identity/homology between amino acid sequences may be calculated using well known scoring matrices such as any one of BLOSUM 30, BLOSUM 40, BLOSUM 45, BLOSUM 50, BLOSUM 55, BLOSUM 60, BLOSUM 62, BLOSUM 65, BLOSUM 70, BLOSUM 75, BLOSUM 80, BLOSUM 85, and BLOSUM 90.

Preferably, the present invention is a nucleic acid construct wherein the encoded at least one invariant chain is a fragment of the SEQ ID NO: 2 of at least 186 amino acids. This includes any of the fragments as defined above, and which thus share identity with the sequence of the invariant chain of SEQ ID NO: 2.

The present invention furthermore relates to a nucleic acid construct wherein the encoded at least one invariant chain is at least 85% identical to SEQ ID NO: 2.

This encompasses that any sequence derived from the invariant chain as put forward in SEQ ID NO: 2 of at least 85 % sequence identity, for example at least 90 % sequence identity, for example at least 91% sequence identity, such as at least 92 % sequence identity, for example at least 93 % sequence identity, such as at least 94 % sequence identity, for example at least 95 % sequence identity, such as at least 96 % sequence identity, for example at least 97% sequence identity, such as at least 98 % sequence identity, for example 99% sequence homology with SEQ ID NO: 2 are included within the scope of the present invention. This includes sequences that are either longer or shorter than the sequence described in SEQ ID NO: 2.

Most preferably, the present invention relates to a nucleic acid construct wherein the encoded at least one invariant chain is identical to SEQ ID NO: 2.

Any of the above described sequences regardless of origin, sequence identity or length are from hereon termed variants of invariant chain.

It follows, that it is within the scope of the present invention that a variant of invariant chain from any organism may be a variant according to the above, i.e. that the variant may be a fragment of the invariant chain of an organism and/or be at least 85% identical to said invariant chain either over all the sequence of the invariant chain or within the fragment of same. The invariant chain may also be from a related species of organism or be from a distantly related species.

Another aspect of the present invention relates to the addition, removal or substitution of regions, peptides or domains of the at least one invariant chain as encoded by the nucleic acid construct. The removal of one or more of these regions, peptides or domains will truncate the resulting invariant chain. The addition or replacement of a region, peptide or domain includes the options of choosing these sequences from known sources such as naturally occurring proteins or polypeptides or from artificially synthesized polypeptides or nucleic acids encoding the same. The addition of regions, domains or peptides includes the option of adding one, two or more of each type or of different types of regions, domains, peptides and one, two, three or more of the nucleic acids encoding these regions, domains and peptides. These may be identical or differ from one another based on the sequence. The regions, peptides and domains need not arise from the same organism as the scaffold invariant chain. It is well known in the art to perform additions, deletions and substitutions of individual as well as stretches of nucleotides which will encode the resulting polypeptide.

Aligning nucleic acid and especially protein sequences of homologous genes or proteins from different organisms can be of great assistance when determining which substitutions, deletions, rearrangements or other alterations it would be beneficial to construct. Aligning human and murine invariant chain sequences as illustrated below, gives an indication of which amino acid residues may be of importance for the structure and function of the invariant chain in these organisms - these are the residues which are conserved between the two sequences. Likewise, the presumably less important residues are the ones in which the sequences differ. It is of interest in regard to the present invention to perform substitutions and/or deletions of the variant residues / regions. When attempting to mutate or delete or otherwise alter the sequence of e.g. the human invariant chain in order to improve its immune response stimulating capacity, it may also be relevant to examine the conserved residues and make e.g. homologous substitutions (i.e. substitutions where the amino acids are considered to be of e.g. same structural quality, polarity, hydrophobicity or other).

A preferred embodiment of the present invention relates to the at least one invariant chain wherein the signal peptide is removed, replaced or added onto the sequence encoding the invariant chain. A signal peptide is a short sequence of amino acids that determine the eventual location of a protein in the cell, also referred to as a sorting peptide. Signal peptides that determine the location of proteins to subcellular compartments such as the endoplasmatic reticulum, golgi apparatus and the various compartments comprising the golgi apparatus, the nucleus, the plasma membrane, mitochondria and the various spaces and membranes herein, peroxisomes, lysosomes, endosomes and secretory vesicles among others are all included within the scope of the present invention. A preferred embodiment comprises alone the lysosomal targeting sequence of invariant chain. Another preferred embodiment comprises alone the KEY region of invariant chain.

Another preferred embodiment of the present invention relates to the removal, addition, or replacement of the CLIP region of the at least one invariant chain. As described above, the addition or replacement of the CLIP region includes the options of adding or replacing the existing CLIP region in the variant of the invariant chain or chains chosen, with CLIP regions from invariant chains of the same or other organisms or of variants of CLIP regions form the same or other organisms. The variant CLIP regions may, as follows from the above, be specifically generated mutant versions of the CLIP region, generated by single or multiple nucleic acid substitutions, deletions or additions. A preferred embodiment comprises the CLIP region alone, or the CLIP region together with the N-terminally adjacent sequence or the C-terminally adjacent sequence without any other regions or domains of invariant chain. Other preferred embodiments comprise alone the N-terminally or C-terminally adjacent sequences to the CLIP region but without the CLIP region itself. By adjacent is meant any amino acids within 10 residues of the CLIP region, within 20 residues, within 30 residues, within 40 residues, within 50 residues, within 75 residues or within 100 residues of the CLIP region.

An embodiment of the present invention relates to fragments of invariant chain as described above without the CLIP region. These fragments may be at least 5 amino acid residues long, at least 10 residues, at least 15 residues, at least 20 residues, at least 25 residues, at least 30 residues or at least 35 residues in length. Another embodiment relates to fragments of invariant chain wherein the signal peptide is removed and the invariant chain fragment is at least 10 amino acid residues long, at least 15 residues, at least 20 residues, at least 25 residues, at least 30 residues, at least 35 residues, at least 50 residues at least 60 residues, at least 70 residues at least 80 residues, at least 90 residues, at least 100 residues, at least 110 residues at least 120 residues at least 130 residues, at least 140 residues, at least 150 residues, at least 160 residues, at least 170 residues, or at least 180 residues in length.

### Antigen

Any of the above variants of invariant chain are encompassed in the present invention in the form wherein at least one of said variants is operatively linked to at least one antigen such as an antigenic protein or peptide or an antigenic fragment of said protein or peptide.

It is an object of the present invention to include but not limit the antigenic proteins or peptides or fragments of said proteins or peptides to stem from pathogenic organisms, cancer-specific polypeptides and antigens, and proteins or peptides associated with an abnormal physiological response.

More preferably it is an object of the present invention to include an antigen originating from any of the following types of pathogens: virus, micro organisms and parasites. This includes pathogens of any animal known. It is preferable to have an antigen from a mammalian pathogen i.e. a pathogen that specifically targets mammalian animals. It is more preferred to have an antigen from a human pathogen. In general, any antigen that is found to be associated with a human pathogen may be used.

In a preferred embodiment at least one antigen may originate from, but is not limited to any of the following families of virus: Adenovirus, arenaviridae, astroviridae, bunyaviridae, caliciviridae, coronaviridae, flaviviridae, herpesviridae, orthomyxoviridae, paramyxoviridae, picomaviridae, poxviridae, reoviridae, retroviridae, rhabdoviridae and togaviridae.

More specifically at least one antigen or antigenic sequence may be derived from any of the following virus: Influenza A such as H1N1, H1N2, H3N2 and H5N1 (bird flu), Influenza B, Influenza C virus, Hepatitis A virus, Hepatitis B virus, Hepatitis C virus, Hepatitis D virus, Hepatitis E virus, Rotavirus, any virus of the Norwalk virus group, enteric adenoviruses, parvovirus, Dengue fever virus, Monkey pox, Mononegavirales, Lyssavirus such as rabies virus, Lagos bat virus, Mokola virus, Duvenhage virus, European bat virus 1 & 2 and Australian bat virus, Ephemerovirus, Vesiculovirus, Vesicular Stomatitis Virus (VSV), Herpesviruses such as Herpes simplex virus types 1 and 2, varicella zoster, cytomegalovirus, Epstein-Bar virus (EBV), human herpesvirusses (HHV), human herpesvirus type 6 and 8, Human immunodeficiency virus (HIV), papilloma virus, murine gammaherpesvirus, Arenaviruses such as Argentine hemorrhagic fever virus, Bolivian hemorrhagic fever virus, Sabia-associated hemorrhagic fever virus, Venezuelan hemorrhagic fever virus, Lassa fever virus, Machupo virus, Lymphocytic choriomeningitis virus (LCMV), Bunyaviridiae such as Crimean-Congo hemorrhagic fever virus, Hantavirus, hemorrhagic fever with renal syndrome causing virus, Rift Valley fever virus, Filoviridae (filovirus) including Ebola hemorrhagic fever and Marburg hemorrhagic fever, Flaviviridae including Kaysanur Forest disease virus, Omsk hemorrhagic fever virus, Tick-borne encephalitis causing virus and Paramyxoviridae such as Hendra virus and Nipah virus, variola major and variola minor (smallpox), alphaviruses such as Venezuelan equine encephalitis virus, eastern equine encephalitis virus, western equine encephalitis virus, SARS-associated coronavirus (SARS-CoV), West Nile virus, any encephaliltis causing virus.

In a preferred embodiment of the invention the at least one antigenic protein or peptide is from a virus selected from the group of: HIV, Hepatitis C virus, influenza virus, herpes virus, Lassa, Ebola, smallpox, Bird flu, filovirus, Marburg, and papilloma virus.

In a more preferred embodiment of the invention the at least one antigenic protein or peptide is selected from the group of and/or may be at least one antigenic fragment of any of the following: vesicular stomatitis virus glycoprotein (VSV-GP), Influenza A NS-1 (non-structural protein 1), Influenza A M1 (matrix protein 1), Influenza A NP (nucleoprotein), LCMV NP, LCMV GP, Ebola GP, Ebola NP, murine gammaherpesvirus M2, M3 and ORF73 (such as MHV-68 M2, M3 and ORF73), chicken Ovalbumin (OVA), or a helper T-cell epitope. It is within the scope of the invention to combine two or more of any of the herein mentioned antigens.

An embodiment of the present invention includes at least one antigenic protein or peptide or fragment of an antigenic protein or peptide from a micro organism. More specifically at least one antigen may be derived from the one of the following from a non-exhaustive list: Anthrax (Bacillus anthracis), Mycobacterium tuberculosis, Salmonella (Salmonella gallinarum, S. pullorum, S. typhi, S. enteridtidis, S. paratyphi, S. dublin, S. typhimurium), Clostridium botulinum, Clostridium perfringens, Corynebacterium diphtheriae, Bordetella pertussis, Campylobacter such as Campylobacter jejuni, Crytococcus neoformans, Yersinia pestis, Yersinia enterocolitica, Yersinia pseudotuberculosis, Listeria monocytogenes, Leptospira species, Legionella pneumophila, Borrelia burgdorferi, Streptococcus species such as Streptococcus pneumoniae, Neisseria meningitides, Haemophilus influenzae, Vibrio species such as Vibrio cholerae O1, V. cholerae non-O1, V. parahaemolyticus, V. parahaemolyticus, V. alginolyticus, V. fumissii, V. carchariae, V. hollisae, V. cincinnatiensis, V. metschnikovii, V. damsela, V. mimicus, V. fluvialis, V. vulnificus, Bacillus cereus, Aeromonas hydrophila, Aeromonas caviae, Aeromonas sobria & Aeromonas veronii, Plesiomonas shigelloides, Shigella species such as Shigella sonnei, S. boydii, S. flexneri, and S. dysenteriae, Enterovirulent Escherichia coli EEC (Escherichia coli - enterotoxigenic (ETEC), Escherichia coli - enteropathogenic (EPEC), Escherichia coli O157:H7 enterohemorrhagic (EHEC), Escherichia coli - enteroinvasive (EIEC)), Staphylococcus species, such as S. aureus and especially the vancomycin intermediate/resistant species (VISA/VRSA) or the multidrug resistant species (MRSA), Shigella species, such as S. flexneri, S. sonnei, S. dysenteriae, Cryptosporidium parvum, Brucella species such as B. abortus, B. melitensis, B.ovis, B. suis, and B. canis, Burkholderia mallei and Burkholderia pseudomallei, Chlamydia psittaci, Coxiella burnetii, Francisella tularensis, Rickettsia prowazekii, Histoplasma capsulatum, Coccidioides immitis.

In a preferred embodiment of the invention the at least one antigenic protein or peptide is from a micro-organism selected from the group of: Mycobacterium tuberculosis, Bacillus anthracis, Staphylococcus species, and Vibrio species.

An embodiment of the invention relates to a nucleic acid construct, wherein the at least one antigenic protein or peptide encoded is from a parasite.

Another embodiment of the present invention relates to a nucleic acid construct comprising combinations of at least two antigenic proteins or peptides from any of the abovementioned pathogens.

Preferably the antigen is derived from, but not limited to, a parasite selected from the group of: Plasmodium species such as Plasmodium malariae, Plasmodium ovale, Plasmodium vivax, Plasmodium falciparum, Endolimax nana, Giardia lamblia, Entamoeba histolytica, Cryptosporidum parvum, Blastocystis hominis, Trichomonas vaginalis, Toxoplasma gondii, Cyclospora cayetanensis, Cryptosporidium muris, Pneumocystis carinii, Leishmania donovani, Leishmania tropica, Leishmania braziliensis, Leishmania mexicana, Acanthamoeba species such as Acanthamoeba castellanii, and A. culbertsoni, Naegleria fowleri, Trypanosoma cruzi, Trypanosoma brucei rhodesiense, Trypanosoma brucei gambiense, Isospora belli, Balantidium coli, Roundworm (Ascaris lumbricoides), Hookworm (Necator Americanus, Ancylostoma duodenal), Pinworm (Enterobius vermicularis), Roundworm (Toxocara canis, Toxocara cati), Heart worm (Dirofilaria immitis), Strongyloides (Stronglyoides stercoralis), Trichinella (Trichinella spiralis), Filaria (Wuchereria bancrofti, Brugia malayi, Onchocerca volvulus, Loa loa, Mansonella streptocerca, Mansonella perstans, Mansonella ozzardi), and Anisakine larvae (Anisakis simplex (herring worm), Pseudoterranova (Phocanema, Terranova) decipiens (cod or seal worm), Contracaecum species, and Hysterothylacium (Thynnascaris species) Trichuris trichiura, Beef tapeworm (Taenia saginata), Pork tapeworm (Taenia solium), Fish tapeworm (Diphyllobothrium latum), and Dog tapeworm (Dipylidium caninum), Intestinal fluke (Fasciolopsis buski), Blood fluke (Schistosoma japonicum, Schistosoma mansoni) Schistosoma haematobium), Liver fluke (Clonorchis sinensis), Oriental lung fluke (Paragonimus westermani), and Sheep liver fluke (Fasciola hepatica), Nanophyetus salmincola and N. schikhobalowi.

In a preferred embodiment of the invention the at least one antigenic protein or peptide is from a parasite selected from the group of: Plasmodium species, Leishmania species, and Trypanosoma species.

An aspect of the present invention relates antigens and/or antigenic sequences derived from diseases or agents that infect domestic animals, especially commercially relevant animals such as pigs, cows, horses, sheep, goats, llamas, rabbits, mink, mice, rats, dogs, cats, poultry such as chicken, turkeys, pheasants and others, fish such as trout, salmon and other farmed species. Examples of diseases or agents here of from which at least one antigen or antigenic sequence may be derived include, but are not limited to: Multiple species diseases such as: Anthrax, Aujeszky's disease, Bluetongue, Brucellosis such as: Brucella abortus, Brucella melitensis or Brucella suis; Crimean Congo haemorrhagic fever, Echinococcosis/hydatidosis, virus of the family Picornaviridae, genus Aphthovirus causing Foot and Mouth disease especially any of the seven immunologically distinct serotypes: A, O, C, SAT1, SAT2, SAT3, Asia1, or Heartwater, Japanese encephalitis, Leptospirosis, New world screwworm (Cochliomyia hominivorax), Old world screwworm (Chrysomya bezziana), Paratuberculosis, Q fever, Rabies, Rift Valley fever, Rinderpest, Trichinellosis, Tularemia, Vesicular stomatitis or West Nile fever; Cattle diseases such as: Bovine anaplasmosis, Bovine babesiosis, Bovine genital campylobacteriosis, Bovine spongiform encephalopathy, Bovine tuberculosis, Bovine viral diarrhoea, Contagious bovine pleuropneumonia, Enzootic bovine leukosis, Haemorrhagic septicaemia, Infectious bovine rhinotracheitis /infectious pustular vulvovaginitis, Lumpky skin disease, Malignant catarrhal fever, Theileriosis, Trichomonosis or Trypanosomosis (tsetse-transmitted); Sheep and goat diseases such as: Caprine arthritis / encephalitis, Contagious agalactia, Contagious caprine pleuropneumonia, Enzootic abortion of ewes (ovine chlamydiosis), Maedi-visna, Nairobi sheep disease, Ovine epididymitis (Brucella ovis), Peste des petits ruminants, Salmonellosis (S. abortusovis), Scrapie, Sheep pox and goat pox; Equine diseases such as: African horse sickness, Contagious equine metritis, Dourine, Equine encephalomyelitis (Eastern), Equine encephalomyelitis (Western), Equine infectious anaemia, Equine influenza, Equine piroplasmosis, Equine rhinopneumonitis, Equine viral arteritis, Glanders, Surra (Trypanosoma evansi) or Venezuelan equine encephalomyelitis; Swine diseases such as: African swine fever, Classical swine fever, Nipah virus encephalitis, Porcine cysticercosis, Porcine reproductive and respiratory syndrome, Swine vesicular disease or Transmissible gastroenteritis; Avian diseases such as: Avian chlamydiosis, Avian infectious bronchitis, Avian infectious laryngotracheitis, Avian mycoplasmosis (M. gallisepticum), Avian mycoplasmosis (M. synoviae), Duck virus hepatitis, Fowl cholera, Fowl typhoid, Highly pathogenic avian influenza this being any Influenzavirus A or B and especially H5N1, Infectious bursal disease (Gumboro disease), Marek's disease, Newcastle disease, Pullorum disease or Turkey rhinotracheitis; Lagomorph and rodent diseases such as: Virus enteritis, Myxomatosis or Rabbit haemorrhagic disease; Fish diseases such as: Epizootic haematopoietic necrosis, Infectious haematopoietic necrosis, Spring viraemia of carp, Viral haemorrhagic septicaemia, Infectious pancreatic necrosis, Infectious salmon anaemia, Epizootic ulcerative syndrome, Bacterial kidney disease (Renibacterium salmoninarum), Gyrodactylosis (Gyrodactylus salaris), Red sea bream iridoviral disease; or other diseases such as Camelpox or Leishmaniosis.

In a preferred embodiment of the invention the at least one antigenic protein or peptide is from Aujeszky's disease, Foot and mouth disease, Vesicular stomatitis virus, Avian influenza or Newcastle disease.

Yet a preferred embodiment of the present invention relates to the at least one antigenic protein or peptide or fragment of said antigenic protein or peptide being an antigenic peptide or protein with at least 85% identity to any of the above described antigens. The homology or identity between amino acids may be calculated by any of the previously mentioned BLOSUM scoring matrices.

An embodiment of the invention relates to a nucleic acid construct, wherein the at least one antigenic protein or peptide or fragment of an antigenic protein or peptide is from a cancer-specific polypeptide or cancer antigen.

Many protein/glycoproteins have been identified and linked to certain types of cancer; these are referred to as cancer specific polypeptides, tumor-associated antigens or cancer antigens. In general, any antigen that is found to be associated with cancer tumors may be used. One way in which cancer specific antigens may be found is by subtraction analyses such as various micro array analyses, such as DNA microarray analysis. Herein the gene expression pattern (as seen in the level of RNA or protein encoded by said genes) between healthy and cancerous patients, between groups of cancerous patients or between healthy and cancerous tissue in the same patient is compared. The genes that have approximately equal expression levels are "subtracted" from each other leaving the genes / gene products that differ between the healthy and cancerous tissue. This approach is known in the art and may be used as a method of identifying novel cancer antigens or to create a gene expression profile specific for a given patient or group of patients. Antigens this identified, both single antigen and the combinations in which they may have been found fall within the scope of the present invention.

Preferably the at least one antigen of the present invention is derived from, but not limited to, a cancer specific polypeptide selected from the group of: MAGE-3, MAGE-1, gp100, gp75, TRP-2, tyrosinase, MART-1, CEA, Ras, p53, B-Catenin, gp43, GAGE-1, BAGE-1, PSA, MUC-1, 2, 3, and HSP-70, TRP-1, gp100/pmel17, .beta.-HCG, Ras mutants, p53 mutants, HMW melanoma antigen, MUC-18, HOJ-1, cyclin-dependent kinase 4 (Cdk4), Caspase 8, HER-2/neu, Human papilloma virus HPV type 6, 11, 16, 18, 31and 33, Bcr-Abl tyrosine kinase, carcinoembryonic antigen (CEA), telomerase, and SV40 Large T.

A preferred embodiment of the invention, the at least one antigenic protein or peptide or fragment of an antigenic protein or peptide is from a cancer-specific polypeptide selected from the group of: p53, HER-2/neu, telomerase, and melanoma antigen.

An embodiment of the invention relates to a nucleic acid construct, wherein the at least one antigenic protein or peptide or fragment of an antigenic protein or peptide is from a polypeptide associated with an abnormal physiological response. Such an abnormal physiological response includes, but is not limited to autoimmune diseases, allergic reactions, cancers and congenital diseases. A non-exhaustive list of examples of hereof includes diseases such rheumatoid arthritis, systemic lupus erythematosus, multiple sclerosis, psoriasis and Crohn's disease.

### Operative linker

An aspect of the present invention relates to the nucleic acid construct wherein the operative link between the invariant chain and the antigenic protein or peptide or fragment of antigenic protein or peptide either is a direct link or a link mediated by a spacer region. By the term operative linker is understood a sequence of nucleotides or amino acid residues that bind together two parts of a nucleic acid construct or chimeric polypeptide in a manner securing the biological processing of the nucleic acid or polypeptide. If the operative linker is a direct link, the two nucleic acids each encoding either an open reading frame or a fragment of an open reading frame are placed immediately adjacent to each other and thereby also in frame. If the operative linker is mediated by a spacer region, a series of nucleotides are inserted between the nucleotides encoding the at least one invariant chain and the at least one antigenic peptide, respectively. It is within the scope of the present invention having a spacer region wherein the spacer region merely is a series of nucleotides linking the at least two elements of the present invention in a manner retaining the open reading frames, or the spacer region may encode one or more signals or separate elements as defined herein below.

In a preferred embodiment the invention comprises an operative linker, wherein the operative linker is a spacer region.

In a more preferred embodiment the invention comprises a spacer region encoding at least one helper epitope for class. II MHC molecules. An example of a helper epitope is an immunogenic determinant such as Diphtheria toxin. Especially Diphtheria toxin B fragment COOH-terminal region has been shown to be immunogenic in mice. Furthermore, HSP70, in part or in whole, as well as other immunogenic peptides, such as influenza viral or immunogenic sequences or peptides with an anchoring motif to HLA class I and class II molecules, also may be encoded in the spacer region of the nucleic acid construct.

In another preferred embodiment the spacer region of the nucleic acid construct encodes at least one protease cleavage site. Cleavage sites of lysosomal proteases such as cathepsins, aspartate proteases and zinc proteases as well as other intracellular proteases fall within the scope of the present invention.

In yet a preferred embodiment the operative linker of the nucleic acid construct may comprise at least one siRNA or miRNA encoding sequence. siRNAs (small interfering RNAs) and miRNAs (microRNAs) target endogenous RNAs, in a sequence-specific manner, for degradation. An siRNA or miRNA encoded within the nucleic acid construct of the present invention may thus be chosen to target an undesirable gene product.

In a more preferred embodiment the operative linker comprises at least one polylinker or multiple cloning site (MCS). Polylinkers and MCS's are series of nucleotides comprising restriction enzyme recognition sequences, i.e. sites where a restriction enzyme cut the DNA in blunt or staggered manner facilitating the subcloning of other fragments / sequences of DNA into the nucleic acid construct. The recognition sequences of the polylinkers / MCS's are typically unique meaning that they are not found elsewhere on the nucleic acid construct. The operative linker may furthermore comprise one or more stop or termination codons that signal the release of the nascent polypeptide from the ribosome. The operative linker may also comprise at least one IRES (internal Ribosomal Entry Site) and / or at least one promoter. An IRES is a nucleotide sequence that allows for translation initiation in the middle of a messenger RNA (mRNA) sequence as part of the greater process of protein synthesis. A promoter is a DNA sequence that enables a gene to be transcribed. The promoter is recognized by RNA polymerase, which then initiates transcription, see in the below. The promoter may be single or bidirectional.

In a very preferred embodiment the operative linker spanning the region between the invariant chain and the at least one antigen is an operative linker comprising at least one polylinker, and at least one promoter, and optionally also at least one IRES. These elements may be placed in any order. In a further preferred embodiment, the STOP codon of the invariant chain has been deleted, and the polylinker has been cloned into the vector in a manner conserving the open reading frame allowing for in frame reading of the at least one antigen that is inserted into the polylinker. This has the advantage of facilitating subcloning of multiple antigens into the same construct in one step or in multiple cloning steps and allowing for the simultaneous expression of multiple antigens in the same frame as the invariant chain. A STOP codon may be inserted after the polylinker for translation termination. This embodiemtn may be combined with any of the above helper epitopes, mi/siRNAs or any of the other elements herein described.

An embodiment of the present invention relates to the placement of the operative linker in relations to the at least one invariant chain and the at least one antigenic protein or peptide or fragment of said protein or peptide, wherein the at antigenic peptide encoding sequences are placed: within the invariant chain sequence, at the front end of the invariant chain sequence, at the terminal part of the invariant chain sequence. This is done in a manner ensuring the readability of the open reading frame of the construct, so that the antigenic peptide is: preceded, surrounded or rounded off by, at least one operative linker.

A preferred embodiment of the present invention further relates to the placement of the operative linker in relations to the at least one invariant chain and the at least one antigenic protein or peptide or fragment of said protein or peptide, wherein the at least one antigenic peptide encoding sequence preferably is placed at the terminal part of the invariant chain and an operative linker is inserted herein between. The terminal part being the first or last residue of the invariant chain or fragment hereof.

### Combinations

It is within the scope of the present invention that the nucleic acid construct encodes a plurality of elements. The elements being the at least one invariant chain and the at least one antigenic protein or peptide or fragment of said protein or peptide. It therefore falls within the scope of the present invention to have a plurality of invariant chains each of these being operatively linked to each other and to a plurality of antigenic proteins or peptides or fragments of antigenic proteins or peptides, wherein these also are operatively linked. The elements of the nucleic acid construct must thus be operatively linked to each other. Several series of invariant chains each operatively linked to one antigenic protein or peptide or fragment of said protein or peptide, each of these series being operatively linked to each other are encompassed within the present invention.

Advantages and very important aspects of the present invention relate to the fact that any type of immune response e.g. T cell mediated and antibody mediated responses, can be initiated, both with epitopes known to be weak antigens, with polypeptides of unknown antigenic properties, and with multiple epitopes/antigens simultaneously.

It is therefore also within the scope of the present invention that a preferred embodiment is a nucleic acid construct encoding at least one invariant chain operatively linked to a plurality of antigenic proteins or peptides or fragment of proteins or peptides, such as two, three, four, five, six, eight, ten, twelve or more antigenic proteins or peptides or fragment of proteins or peptides.
The nucleic acid construct may comprise additional elements. These include but are not limited to: internal ribosomal entry sites (IRES), genes encoding proteins related to antigen presentation such as LAMP, calreticulin and Hsp70, genes encoding proteins that are related to intracellular spreading such as VP22, HIV Tat, Cx43 or other connexins and intercellular gap-junction constituents, genes encoding natural killer cell (NK-cell) activation molecules such as H60 and cytokines, chicken ovalbumin, or any T-helper cell epitope.

In a preferred embodiment of the present invention the nucleic acid construct comprises at least one gene encoding a protein related to antigen presentation such as LAMP, LIMP, calreticulin or Hsp70.

In yet a preferred embodiment of the present invention the nucleic acid construct comprises at least one gene encoding a protein related to intracellular spreading such as VP22, Cx43, HIV Tat, other connexins or intercellular gap-junction constituents.

### Promoter

The term promoter will be used here to refer to a group of transcriptional control modules that are clustered around the initiation site for RNA polymerase II. Much of the thinking about how promoters are organized derives from analyses of several viral promoters, including those for the HSV thymidine kinase (tk) and SV40 early transcription units. These studies, augmented by more recent work, have shown that promoters are composed of discrete functional modules, each consisting of approximately 7-20 bp of DNA, and containing one or more recognition sites for transcriptional activator proteins. At least one module in each promoter functions to position the start site for RNA synthesis. The best known example of this is the TATA box, but in some promoters lacking a TATA box, such as the promoter for the mammalian terminal deoxynucleotidyl transferase gene and the promoter for the SV 40 late genes, a discrete element overlying the start site itself helps to fix the place of initiation.

Additional promoter elements regulate the frequency of transcriptional initiation. Typically, these are located in the region 30-110 bp upstream of the start site, although a number of promoters have recently been shown to contain functional elements downstream of the start site as well. The spacing between elements is flexible, so that promoter function is preserved when elements are inverted or moved relative to one another. In the tk promoter, the spacing between elements can be increased to 50 bp apart before activity begins to decline. Depending on the promoter, it appears that individual elements can function either cooperatively or independently to activate transcription. Any promoter that can direct transcription initiation of the sequences encoded by the nucleic acid construct may be used in the invention.

An aspect of the present invention comprises the nucleic acid construct wherein the at least one operatively linked invariant chain and antigenic protein or peptide encoding sequence is preceded by a promoter enabling expression of the construct.

It is a further aspect that the promoter is selected from the group of constitutive promoters, inducible promoters, organism specific promoters, tissue specific promoters and cell type specific promoters.

Examples of promoters include, but are not limited to: constitutive promoters such as: simian virus 40 (SV40) early promoter, a mouse mammary tumor virus promoter, a human immunodeficiency virus long terminal repeat promoter, a Moloney virus promoter, an avian leukaemia virus promoter, an Epstein-Barr virus immediate early promoter, a Rous sarcoma virus (RSV) promoter, a human actin promoter, a human myosin promoter, a human haemoglobin promoter, cytomegalovirus (CMV) promoter and a human muscle creatine promoter, inducible promoters such as: a metallothionine promoter, a glucocorticoid promoter, a progesterone promoter, and a tetracycline promoter (tet-on or tet-off), tissue specific promoters such as: HER-2 promoter and PSA associated promoter and bidirectional promoters, that are capable of initiating transcription in either direction from the promoter.

Advantages of using an inducible promoter includes the option of providing a "dormant" vaccine that can be activated at will. This may be of use if the vaccination preferably only is induced locally vs. systemically within a body (e.g. in cases involving cancer), or the vaccine is detrimental to the health of the recipient at the time of vaccination.

In a preferred embodiment the nucleic acid construct comprises a promoter selected from the group of: CMV promoter, SV40 promoter and RSV promoter.

### Delivery vehicle

An aspect of the present invention comprises the nucleic acid construct as described in any of the above, comprised within a delivery vehicle. A delivery vehicle is an entity whereby a nucleotide sequence or polypeptide or both can be transported from at least one media to another. Delivery vehicles are generally used for expression of the sequences encoded within the nucleic acid construct and/or for the intracellular delivery of the construct or the polypeptide encoded therein. It is within the scope of the present invention that the delivery vehicle is a vehicle selected from the group of: RNA based vehicles, DNA based vehicles/ vectors, lipid based vehicles, virally based vehicles and cell based vehicles. Examples of such delivery vehicles include, but are not limited to: biodegradable polymer microspheres, lipid based formulations such as liposome carriers, coating the construct onto colloidal gold particles, lipopolysaccharides, polypeptides, polysaccharides, and pegylation of viral vehicles.

A preferred embodiment of the present invention regards delivery of the nucleic acid construct as naked DNA by mechanical or electrical techniques. Especially the coating of the nucleic acid construct upon gold particles is a favoured embodiment. The delivery of the nucleic acid construct upon gold particles is done by ballistic transfer using particle bombardment equipment such as a gene gun.

A more preferred embodiment of the present invention comprises a virus as a delivery vehicle, where the virus is selected from the non-exhaustive group of: adenoviruses, retroviruses, lentiviruses, adeno-associated viruses, herpesviruses, vaccinia viruses, foamy viruses, cytomegaloviruses, Semliki forest virus, poxviruses, RNA virus vector and DNA virus vector. Such viral vectors are well known in the art.

Viral vectors are often made up of two components, a modified viral genome and a coat structure surrounding it, although sometimes viral vectors are introduced in naked form or coated with proteins other than viral proteins. Most current vectors have coat structures similar to a wild-type virus. This structure packages and protects the viral nucleic acid and provides the means to bind and enter target cells.

Preferably, viral vectors are modified from wild-type viral genomes to disable the growth of the virus in a target cell while enabling the virus to grow in a host cell (e.g. such as a packaging or helper cell) used to prepare infectious particles. Vector nucleic acids generally essential cis-acting viral sequences for replication and packaging in a helper line and expression control sequences for regulating the expression of a polynucleotide being delivered to a target cell. Other viral functions are expressed in trans in specific packaging or helper cell lines as known in the art.

### Adenovirus

In a more preferred embodiment the vehicle comprising the nucleic acid construct as described herein is an adenovirus. The adenoviral genome consists of a linear double-stranded DNA molecule of approximately 36 kb carrying more than about thirty genes necessary to complete the viral replication cycle. The early genes are divided into 4 regions (E1 to E4) that are essential for viral replication with the exception of the E3 region, which is believed to modulate the anti-viral host immune response. The E1 region (EIA and EIB) encodes proteins responsible for the regulation of transcription of the viral genome. Expression of the E2 region genes (E2A and E2B) leads to the synthesis of the polypeptides needed for viral replication. The proteins encoded by the E3 region prevent cytolysis by cytotoxic T cells and tumor necrosis factor. The proteins encoded by the E4 region are involved in DNA replication, late gene expression and splicing and host cell shut off. The late genes generally encode structural proteins contributing to the viral capsid. In addition, the adenoviral genome carries at cis-acting 5' and 3' ITRs (Inverted Terminal Repeat) and packaging sequences essential for DNA replication. The ITRs harbor origins of DNA replication while the encapsidation region is required for the packaging of adenoviral DNA into infectious particles (see for example US 2004/0157307).

In the most preferred embodiment of the present invention the vehicle comprising the nucleic acid construct as described herein is a replication defective adenovirus or a conditionally replication deficient adenovirus. Adenoviral vectors can be engineered to be conditionally replicative (CRAd vectors) in order to replicate selectively in specific cells (e.g., such as proliferative cells). In another aspect, an adenoviral vector is replication-defective for the E1 function (e.g., by total or partial deletion or mutagenesis of E1). The adenoviral backbone of the vector may comprise additional modifications (deletions, insertions or mutations in one or more viral genes). An example of an E2 modification is illustrated by the thermosensitive mutation localized on the DBP (DNA Binding Protein) encoding gene. The adenoviral sequence may also be deleted of all or part of the E4 region. Additional deletions within the non-essential E3 region may allow the size of the polynucleotide being delivered to be increased. However, it may be advantageous to retain all or part of the E3 sequences coding for polypeptides (e.g., such as gp19k) allowing the virus to escape the immune system or inflammatory reactions. Second generation vectors retaining the ITRs and packaging sequences and comprising substantial genetic modifications to abolish the residual synthesis of the viral antigens also may be used in order to improve long-term expression of the expressed gene in the transduced cells. The nucleic acid construct being introduced into the cell may be inserted in any location of the viral genome, with the exception of the cis-acting sequences (see for example US 2004/0157307).

Adenoviruses can be derived from any human or animal source, in particular canine, avian, bovine, murine, ovine, feline, porcine or simian sources or alternatively, may be a hybrid virus. Any serotype can be employed. However, the human adenoviruses are preferred and such viruses are available, for example, from the ATCC (American Type Culture Collection).

A preferred embodiment of the present invention comprises an adenovirus such as: Ovine adenovirus, Canine adenovirus type II, Modified vaccinia Ankara (MVA) or MVA-BN.

Adenoviral particles or empty adenoviral capsids also can be used to transfer nucleic acid constructs or nucleic acid based delivery vectors by a virus-mediated co-internalization process. This process can be accomplished in the presence of cationic agent(s) such as polycarbenes or lipid vesicles comprising one or more lipid layers.

Adenoviral particles may be prepared and propagated according to any conventional technique in the field of the art using a complementation cell line or a helper virus, which supplies in trans the missing viral genes necessary for viral replication. The adenoviral particles can be recovered from the culture supernatant but also from the cells after lysis and optionally further purified according to standard techniques (e.g. chromatography and ultracentrifugation).

Cell-type specific targeting may be achieved with vectors derived from adenoviruses having a broad host range by the modification of viral surface proteins. For example, the specificity of infection of adenoviruses is determined by the attachment to cellular receptors present at the surface of permissive cells. In this regard, the fiber and penton present at the surface of the adenoviral capsid play a critical role in cellular attachment. Thus, cell targeting of adenoviruses can be carried out by genetic modification of the viral gene encoding fiber and/or penton, to generate modified fiber and/or penton capable of specific interaction with unique cell surface receptors.

An aspect of the present invention relates to an adenoviral vector comprising a nucleotide construct encoding at least one antigen and at least one protein or peptide or fragment of a protein or peptide which stimulates an MHC-I response.

A further aspect of the present invention relates to an adenoviral vector, wherein the nucleotide construct encodes at least one protein or peptide or fragment of a protein or peptide which stimulates an MHC-II response.

Preferably, the adenoviral vector comprises sequences, wherein the at least one antigen is operatively linked to the at least one MHC response stimulating protein or peptide or fragment of an MHC response stimulating protein or peptide. The MHC stimulating protein or peptide or fragment of protein or peptide is preferably an MHC associated protein or peptide. Such an MHC associated peptide can be but is not limited being selected from the group of: ER localizing peptide, Golgi localizing peptide, endosomal peptide loading compartment localizing peptide, lysosomal, MIIC, CIIV, melanosomes, secretory granules, Birbeck granules.

More preferably the adenoviral vector comprises an endosomal peptide loading compartment localizing peptide. Such an endosomal peptide loading compartment localizing peptide can be, but is not limited to being, selected from the group of: sorting signal peptides, LAMP, LIMP and invariant chain.

Most preferably the adenoviral vector comprises at least one MHC response stimulating protein or peptide or fragment of protein or peptide and said MHC response stimulating protein or peptide or fragment of protein or peptide is invariant chain.

The adenoviral vector may furthermore comprise proteins that assist in the spreading of the virus or the construct comprised therein. Such proteins include connexins, gap-junction related proteins and pore-forming proteins. A preferred embodiment of the present invention comprises an adenoviral vector encoding or otherwise comprising any one or more of the following proteins related to intercellular spreading: VP22, Cx43 and HIV Tat.

### Recombinant cell

An aspect of the present invention relates to a cell comprising the nucleic acid construct as defined in any of the above. Such a recombinant cell can be used a tool for in vitro research, as a delivery vehicle for the nucleic acid construct or as part of a gene therapy regime. The nucleic acid construct and nucleic acid based vectors according to the invention can be introduced into cells by techniques well known in the art and which include microinjection of DNA into the nucleus of a cell, transfection, electroporation, lipofection/liposome fusion and particle bombardment. Suitable cells include autologous and non-autologous cells, and may include xenogenic cells.

In a preferred embodiment the nucleic acid construct of the present invention is comprised within an antigen presenting cell (APC). Any cell that presents antigens on its surface in association with an MHC molecule is considered an antigen presenting cell. Such cells include but are not limited to macrophages, dendritic cells, B cells, hybrid APCs, and foster APCs. Methods of making hybrid APCs are well known in the art.

In a more preferred embodiment the APC is a professional antigen presenting cell and most preferably the APC is an MHC-I and/or MHC-II expressing cell.

The APC according to any of the above may be a stem cell obtained from a patient. After introducing the nucleic acid construct of the invention, the stem cell may be reintroduced into the patient in an attempt to treat the patient of a medical condition. Preferably, the cell isolated from the patient is a stem cell capable of differentiating into an antigen presenting cell.

It is furthermore included within the scope of the present invention to that the antigen presenting cell comprising the nucleic acid construct of the present invention does not express any co-stimulatory signals and the antigenic protein or peptide or antigenic fragment of said protein or peptide is an auto-antigen.

### Chimeric Proteins and antibodies

An object of the present invention is the chimeric protein encoded by the nucleic acid constructs as described herein above, comprising at least one operatively linked invariant chain and at least one antigenic protein or peptide or fragment of said antigenic protein or peptide. By chimeric protein is understood a genetically engineered protein that is encoded by a nucleotide sequence made by a splicing together of two or more complete or partial genes or a series of (non)random nucleic acids.

An aspect of the present invention relates to an antibody that can recognize the chimeric protein as defined herein above. By the term antibody is understood immunoglobulin molecules and active portions of immunoglobulin molecules. Antibodies are for example intact immunoglobulin molecules or fragments thereof retaining the immunologic activity. Such antibodies can be used for the passive immunization of an animal, or for use in an assay for detecting proteins to which the antibody binds.

### Vaccine compositions

An aspect of the present invention relates to a vaccine comprising a nucleic acid sequence encoding at least one invariant chain operatively linked to at least one antigenic protein or peptide or fragment of said antigenic protein or peptide. The vaccine may thus comprise a nucleic acid construct as defined in any of the above. The vaccine may furthermore be used as a medicament.

The vaccine composition according to the invention can be formulated according to known methods such as by the admixture of one or more pharmaceutically acceptable carriers, also known as excipients or stabilizers with the active agent. These excipients may be acceptable for administration to any individual / animal, preferably to vertebrates and more preferably to humans as they are non-toxic to the cell or individual being exposed thereto at the dosages and concentrations employed. Often the physiologically acceptable carrier is an aqueous pH buffered solution. Examples of such excipients, carriers and methods of formulation may be found e.g. in Remington's Pharmaceutical Sciences (Maack Publishing Co, Easton, PA). Examples of physiologically acceptable carriers include but are not limited to: buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN.TM., polyethylene glycol (PEG), and PLURONICS.TM.

To formulate a pharmaceutically acceptable composition suitable for effective administration, such compositions will according to the invention contain an effective amount of the nucleic acid construct, the nucleic acid construct comprised within a delivery vehicle or the chimeric protein encoded within the nucleic acid construct as described herein. Often, if vaccinating with protein or polypeptides as encoded by the nucleic acid construct of the present invention, a carrier will be used as a scaffold by coupling the proteins or peptides hereto and thus aiding in the induction of an immune response. The carrier protein may be any conventional carrier including any protein suitable for presenting immunogenic determinants. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and inactive virus particles. Such carriers are well known to those of ordinary skill in the art. Additionally, these carriers may function as immunostimulating agents ("adjuvants"). Immunisation of the animal may be carried out with adjuvants and/or pharmaceutical carriers. Conventional carrier proteins include, but are not limited to, keyhole limpet hemocyanin, serum proteins such as transferrin, bovine serum albumin, or human serum albumin, an ovalbumin, immunoglobulins, or hormones, such as insulin. The carrier may be present together with an adjuvant or independently here from.

In the following vaccine compositions are meant to encompass compositions useful for prophylactic and therapeutic use, including stimulating an immune response in a patient. It is further contemplated that the vaccine composition of the invention does not induce any systemic or local toxicity reactions or any other side effects.

In a preferred embodiment the nucleic acid construct of the vaccine is packaged. Packaging means for the nucleic acid construct include means selected from, but not limited to the group of: RNA based or DNA based vectors, lipid based carriers, viral expression vectors, viral delivery vectors, cell based vehicles, coating of colloidal gold particles, biodegradable polymer microspheres. Any of the previously mentioned delivery means may thus be used for packing purposes for use in a vaccine composition.

In a more preferred embodiment the packaging means of the nucleic acid construct for the vaccine is a viral expression vector selected from, but not limited to the group of: adenovirus, retrovirus, lentivirus, adeno-associated virus, herpes virus, vaccinia virus and DNA virus vector.

In an even more preferred embodiment the nucleic acid construct of the vaccine is packaged into an adenoviral vector. In the most preferred embodiment the nucleic acids construct as described in any of the herein above of the vaccine is packaged in a replication deficient or conditionally replication deficient adenoviral vector. Adenoviral vectors are described in detail in the above.

An aspect of the invention relates to a vaccine comprising at least two vectors. This encompasses that any one or two different nucleic acid constructs as described may be packed into at least two vectors, these vectors being of a type as described in any of the above. The invention furthermore relates to a vaccine comprising three, four, five or six vectors. Again, these vectors may differ from one another or not, and may carry identical or different nucleic acid constructs as described herein above. A further aspect of the present invention relates to a vaccine comprising at least one chimeric protein as encoded by any of the nucleic acid constructs described herein. When a chimeric protein or polypeptide is to be used as an immunogen, it may be produced by expression of any one or more of the DNA constructs described above in a recombinant cell or it may be prepared by chemical synthesis by methods known in the art. As described in the above, such chimeric proteins and / or peptides may be coupled to carriers to increase the immunologic response to the proteins /peptides and may be administered with or without an adjuvant and/or excipient.

### Adjuvant

Adjuvants may be included in the vaccine composition to enhance the specific immune response. Thus, it is particular important to identify an adjuvant that when combined with the antigen(s) / nucleic acid constructs and / or delivery vehicles such as adenoviral vehicles (any of which may also be referred to as immunogenic determinant), results in a vaccine composition capable of inducing a strong specific immunological response. The immunogenic determinant may also be mixed with two or more different adjuvants prior to immunisation. Vaccine compositions are also referred to as immunogenic compositions in the present text.

A large number of adjuvants have been described and used for the generation of antibodies in laboratory animals, such as mouse, rats and rabbits. In such setting the tolerance of side effect is rather high as the main aim is to obtain a strong antibody response. For use and for approval for use in pharmaceuticals, and especially for use in humans it is required that the components of the vaccine composition, including the adjuvant, are well characterized. It is further required that the composition has minimal risk of any adverse reaction, such as granuloma, abscesses or fever.

An embodiment of the present invention relates to a vaccine comprising an adjuvant. In a preferred embodiment the vaccine composition is suitable for administration to a mammal, and most preferably to a human subject. Therefore the preferred adjuvant is suitable for administration to a mammal and most preferably is suitable for administration to a human subject.

The choice of adjuvant may further be selected by its ability to stimulate the type of immune response desired, B-cell or/and T-cell activation and the vaccine composition may be formulated to optimize distribution and presentation to the relevant lymphatic tissues.

Adjuvants pertaining to the present invention may be grouped according to their origin, be it mineral, bacterial, plant, synthetic, or host product. The first group under this classification is the mineral adjuvants, such as aluminum compounds. Antigens precipitated with aluminum salts or antigens mixed with or adsorbed to performed aluminum compounds have been used extensively to augment immune responses in animals and humans. Aluminium particles have been demonstrated in regional lymph nodes of rabbits seven days following immunization, and it may be that another significant function is to direct antigen to T cell containing areas in the nodes themselves. Adjuvant potency has been shown to correlate with intimation of the draining lymph nodes. While many studies have confirmed that antigens administered with aluminium salts lead to increased humoral immunity, cell mediated immunity appears to be only slightly increased, as measured by delayed-type hypersensitivity. Aluminium hydroxide has also been described as activating the complement pathway. This mechanism may play a role in the local inflammatory response as well as immunoglobulin production and B cell memory. Furthermore, aluminum hydroxide can protect the antigen from rapid catabolism. Primarily because of their excellent record of safety, aluminum compounds are presently the only adjuvants used in humans.

Another large group of adjuvants is those of bacterial origin. Adjuvants with bacterial origins can be purified and synthesized (e.g. muramyl dipeptides, lipid A) and host mediators have been cloned (Interleukin 1 and 2). The last decade has brought significant progress in the chemical purification of several adjuvants of active components of bacterial origin: Bordetella pertussis, Mycobacterium tuberculosis, lipopoly-saccharide, Freund's Complete Adjuvant (FCA) and Freund's Incomplete Adjuvant (Difco Laboratories, Detroit, Mich.) and Merck Adjuvant 65 (Merck and Company, Inc., Rahway, N.J.). Additionally suitable adjuvants in accordance with the present invention are e.g. Titermax Classical adjuvant (SIGMA-ALDRICH), ISCOMS, Quil A, ALUN, see US 58767 and 5,554,372, Lipid A derivatives, choleratoxin derivatives, HSP derivatives, LPS derivatives, synthetic peptide matrixes, GMDP, and other as well as combined with immunostimulants (US 5,876,735). B. pertussis is of interest as an adjuvant in the context of the present invention due to its ability to modulate cell-mediated immunity through action on T-lymphocyte populations. For lipopolysaccharide and Freund's Complete Adjuvant, adjuvant active moieties have been identified and synthesized which permit study of structure-function relationships. These are also considered for inclusion in immunogenic compositions according to the present invention.

Lipopolysaccharide and its various derivatives, including lipid A, have been found to be powerful adjuvants in combination with liposomes or other lipid emulsions. It is not yet certain whether derivatives with sufficiently low toxicity for general use in humans can be produced. Freund's Complete Adjuvant is the standard in most experimental studies.

Mineral oil may be added to the immunogenic composition in order to protect the antigen from rapid catabolism.

Many other types of materials can be used as adjuvants in immunogenic compositions according to the present invention. They include plant products such as saponin, animal products such as chitin and numerous synthetic chemicals.

Adjuvants according to the present invention can also been categorized by their proposed mechanisms of action. This type of classification is necessarily somewhat arbitrary because most adjuvants appear to function by more than one mechanism. Adjuvants may act through antigen localization and delivery, or by direct effects on cells making up the immune system, such as macrophages and lymphocytes. Another mechanism by which adjuvants according to the invention enhance the immune response is by creation of an antigen depot. This appears to contribute to the adjuvant activity of aluminum compounds, oil emulsions, liposomes, and synthetic polymers. The adjuvant activity of lipopolysaccharides and muramyl dipeptides appears to be mainly mediated through activation of the macrophage, whereas B. pertussis affects both macrophages and lymphocytes. Further examples of adjuvants that may be useful when incorporated into immunogenic compositions according to the present invention are described in US 5,554,372.

Adjuvants useful in both prophylactic and therapeutic vaccines according to the present invention may thus be mineral salts, such as aluminium hydroxide and aluminium or calcium phosphates gels, oil emulsions and surfactant based formulations such as MF59 (microfluidized detergent stabilized oil in water emulsion), QS21 (purified saponin), AS02 (SBAS2, oil-in-water emulsion + monophosphoryl lipid A (MPL) + QS21), Montanide ISA 51 and ISA-720 (stabilized water in oil emulsion), Adjuvant 65 (containing peanut oil, mannide monooleate and aluminum monostearate), RIBI ImmunoChem Research Inc., Hamilton, Utah), particulate adjuvants, such as virosomes (unilamellar liposomal cehicles incorporating influenza haemagglutinin), AS04 (Al salt with MPL), ISCOMS (structured complex of saponins and lipids (such as cholesterol), polyactide co-glycolide (PLG), microbial derivatives (natural and synthetic) such as monophosphoryl lipid A (MPL), Detox (MPL + *M. Phlei* cell wall skeleton), AGP (RC-529 (synthetic acylated monosaccharide)), DC_chol (lipoidal immunostimulators able to self organise into liposomes), OM-174 (lipid A derivative), CpG motifs (synthetic oligonucleotides containing immunostimulatory CpG motifs), modified bacterial toxins, LT and CT, with non-toxic adjuvant effects, Endogenous human immunomodulators, e.g., hGM-CSF or hIL-12 or Immudaptin (C3d tandem array), inert vehicles such as gold particles.

Additional examples of adjuvants comprise: Immunostimulatory oil emulsions (for example, water-in-oil, oil-in-water, water-in-oil-in-water such as e.g. Freund's incomplete adjuvant such as Montainde®, Specol, mineral salts such e.g. as Al(OH)₃, AlPO₄, microbial products, Saponins such as Qual A, synthetic products, as well as adjuvant formulations, and immune stimulatory complexes (ISCOMs) and cytokines, heat-inactivated bacteria/components, nanobeads, LPS, LTA. A list of other commonly used adjuvants is disclosed on pages 6-8 in WO 03089471, the list being hereby incorporated by reference.

Immunogenic compositions according to the invention may also contain diluents such as buffers, antioxidants such as ascorbic acid, low molecular weight (less than about 10 residues) polypeptides, proteins, amino acids, carbohydrates including glucose, sucrose or dextrins, chelating agents such as EDTA, glutathione and other stabilizers and excipients. Neutral buffered saline or saline mixed with non-specific serum albumin are exemplary appropriate diluents.

Adjuvants are generally included in the immunogenic compositions in an amount according to the instructions of the manufacturer.

### Administration

Vaccine compositions according to the invention may be administered to an individual in therapeutically effective amounts. The effective amount may vary according to a variety of factors such as the individual's condition, weight, sex and age. Other factors include the mode of administration.

The pharmaceutical or veterinary compositions may be provided to the individual by a variety of routes such as subcutaneous, topical, oral and intramuscular. Administration of pharmaceutical compositions is accomplished orally or parenterally. Methods of parenteral delivery include topical, intra-arterial (directly to the tissue), intramuscular, subcutaneous, intramedullary, intrathecal, intraventricular, intravenous, intraperitoneal, or intranasal administration. The present invention also has the objective of providing suitable topical, oral, systemic and parenteral pharmaceutical formulations for use in the methods of prophylaxis and treatment with the vaccine composition.

For example, the vaccine compositions can be administered in such oral dosage forms as tablets, capsules (each including timed release and sustained release formulations), pills, powders, granules, elixirs, tinctures, solutions, suspensions, syrups and emulsions, or by injection. Likewise, they may also be administered in intravenous (both bolus and infusion), intraperitoneal, subcutaneous, topical with or without occlusion, or intramuscular form, all using forms well known to those of ordinary skill in the pharmaceutical arts. An effective but non-toxic amount of the vaccine, comprising any of the herein described compounds can be employed as a prophylactic or therapeutic agent. Also any and all conventional dosage forms that are known in the art to be appropriate for formulating injectable immunogenic peptide composition are encompassed, such as lyophilized forms and solutions, suspensions or emulsion forms containing, if required, conventional pharmaceutically acceptable carriers, diluents, preservatives, adjuvants, buffer components, etc.

Preferred modes of administration of the vaccine composition according to the invention include, but are not limited to systemic administration, such as intravenous or subcutaneous administration, intradermal administration, intramuscular administration, intranasal administration, oral administration, rectal administration, vaginal administration, pulmonary administration and generally any form of mucosal administration. Furthermore, it is within the scope of the present invention that the means for any of the administration forms mentioned in the herein are included in the present invention.

A vaccine according to the present invention can be administered once, or any number of times such as two, three, four or five times. Administering the vaccine more than once has the effect of boosting the resulting immune response. The vaccine can further be boosted by administering the vaccine in a form or body part different from the previous administration. The booster shot is either a homologous or a heterologous booster shot. A homologous booster shot is a where the first and subsequent vaccinations comprise the same constructs and more specifically the same delivery vehicle especially the same viral vector. A heterologous booster shot is where identical constructs are comprised within different viral vectors. This is especially of interest when employing adenoviral delivery as the human body raises an immune response against a given adenovirus if it has previously been exposed thereto. A preferred embodiment of the present invention therefore relates to the pegylation of the adenoviral vector providing the option of boosting with the same (homologous) adenoviral vector. An alternative and preferred embodiment relates to the sequential boosting of a vaccine with different adenoviral vectors comprising the same constructs.

A preferred administration form of the vaccine according to the present invention is administering the vaccine to the body area, inside or out, most likely to be the receptacle of a given infection. The receptacle of infection is the body area that the infection is received by, e.g. regarding influenza, the receptacle of infection is the lungs.

The vaccine of the present invention can be administered to any organism to which it may be beneficial, especially any animal such as a vertebrate animal. It falls within the scope of the present invention that the means and modes of administration of the vaccine are adapted to the recipient. A preferred recipient of the vaccine is a mammal and the mammal is in a more preferred embodiment of the present invention selected from the group of: cows, pigs, horses, sheep, goats, llamas, mice, rats, monkeys, dogs, cats and humans. In the most preferred embodiment the mammal is a human.

An embodiment of the present invention includes a vaccine composition further comprising a second active ingredient. The second active ingredient is selected from, but not limited the group of antibiotics, chemotherapeutics, anti-allergenics, cytokines, complement factors and co-stimulatory molecules of the immune system.

Another embodiment of the present invention comprises a kit of parts, wherein the kit includes at least one vaccine composition according to any of the above, a means for administering said vaccine and the instruction on how to do so. It is within the scope of the present invention to include multiple dosages of the same vaccine or several different vaccines. In a preferred embodiment the kit of parts further comprises a second active ingredient.

The present invention further comprises a method for inducing an immune response in an animal, comprising administering to the animal a vaccine according to any of the above. The immune response is, but is not limited to any of the following types of responses: an MHC-I dependent response, an MHC-I and/ or MHC-II dependent response, a T-cell dependent response, a CD4 T-cell dependent response, a CD4⁺ T cell independent response, a CD8⁺ T-cell dependent response and a B cell dependent immune response. Another method falling within the scope of the present invention is the method of providing at least one vaccine according to any of the above and administering said at least one vaccine to a subject at least once for treatment or prophylaxis of an animal. The invention also encompasses the nucleic acid construct according the herein described for the preparation of a composition for the production of a vaccine. Said vaccine can be but is not limited being used for genetic immunization of an animal, or to treat a clinical condition in an individual in need thereof.

### Detailed description of the drawings

**Figure 1****:** Schematic drawing of inserts in the adenovirus vector. A) Schematic drawing of Ad-GP expression cassette, B) Schematic drawing of Ad-liGP expression cassette. Shown is also the situation of various LCMV GP epitopes. Ad-GP: adenoviral-glycoprotein, CMV: Cytomegalovirus promoter, li : Invariant chain, LCMV GP 1-498 : Glycoprotein from lymfocytic choriomeningitis virus, STOP : Stop codon, PolyA : SV40 polyadenylation signal

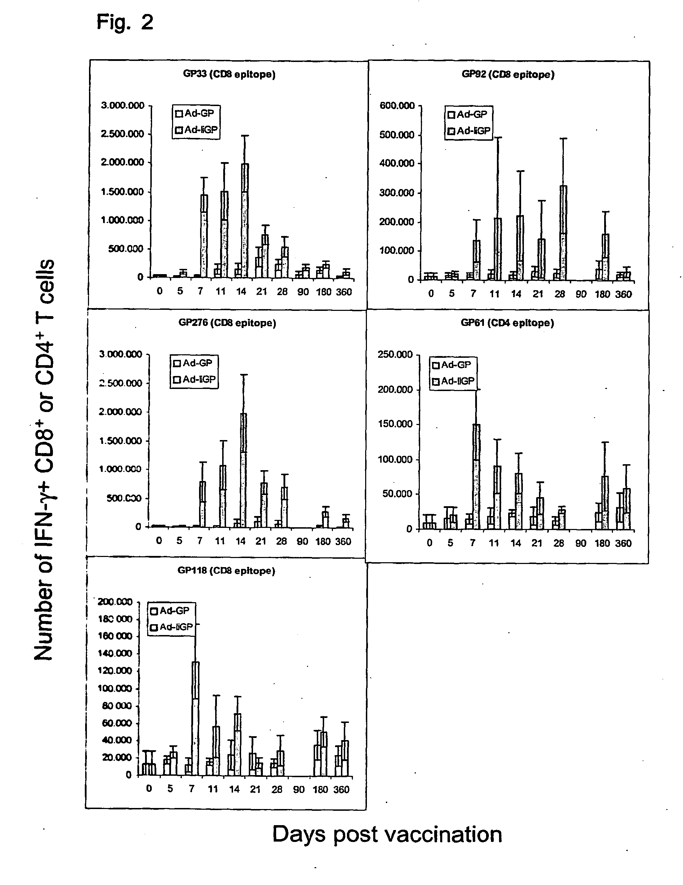

**Figure 2**: CD8⁺ and CD4⁺ T-cell responses to adenovirus encoded epitopes. C57BL/6 mice were vaccinated with 2x10⁷ infectious unit (IFU) of Ad-GP or Ad-liGP in the right hind footpad. On the indicated days post vaccination the number of epitope specific CD8⁺ or CD4⁺ T cells were determined by intracellular staining for peptide-induced IFN-γ of spleen cells. Bars represent Average (Avg) ± standard deviation (SD) of 3-5 animals.

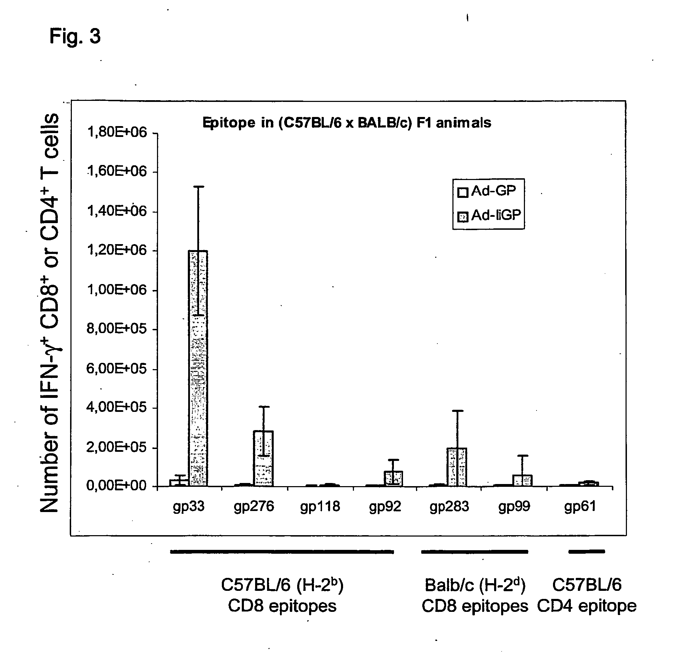

**Figure 3****:** CD8⁺ and CD4⁺ T-cell responses to adenovirus encoded epitopes in F₁ hybrid mice. C57BL/6 x BALB/c (H-2^{bxd}) F₁ mice were vaccinated with 2x10⁷ IFU of Ad-GP or Ad-liGP in the right hind footpad. On day 21 post vaccination the number of epitope specific CD8⁺ or CD4⁺ T cells were determined by intracellular staining for peptide-induced IFN-γ of spleen cells. Bars represent Avg ± SD of 4-5 animals

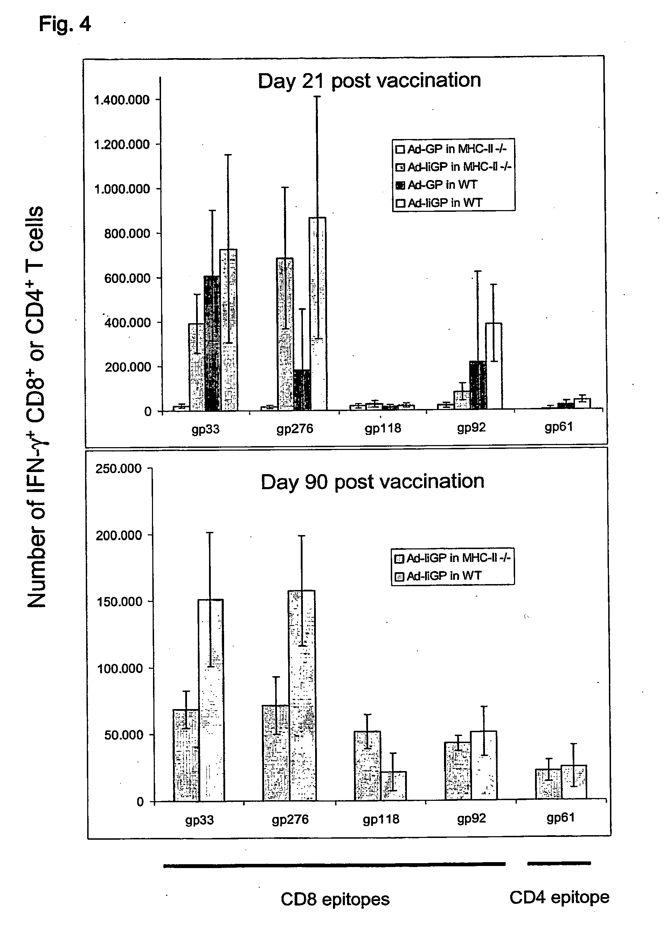

**Figure 4****:** Ad-liGP exerts CD8⁺ T-cell stimulatory effects that are independent of CD4⁺ T-cells. MHC-II^{-/-} or C57BU6 mice were vaccinated with 2x10⁷ IFU of Ad-GP or Ad-liGP in the right hind footpad. On day 21 or 90 post vaccination the number of epitope specific CD8⁺ or CD4⁺ T cells were determined by intracellular staining for peptide-induced IFN-γ of spleen cells. Bars represent Avg ± SD of 4 animals.

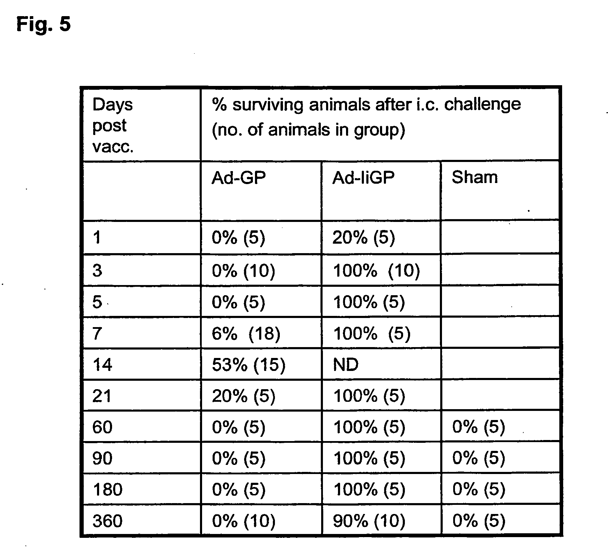

**Figure 5****:** Ad-liGP confers rapid and superior protection against lethal LCMV infection. C57BU6 mice were vaccinated with 2x10⁷ IFU of Ad-GP or Ad-liGP in the right hind footpad. On the indicated days post vaccination animals were challenged with 20 pfu (plaque forming units) LCMV Arm 53b i.c. (intra cerebral). Mortality was recorded for 14 days. Each group consisted of 5 to 18 animals. ND means no data.

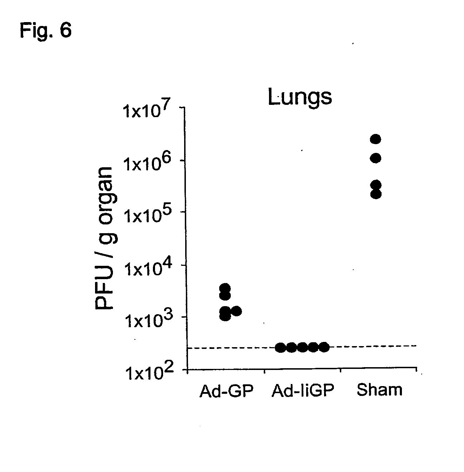

**Figure 6****:** Ad-liGP efficiently protects against high-dose, intravenous LCMV infection. C57BU6 mice were vaccinated with 2x10⁷ IFU of Ad-GP or Ad-liGP in the right hind footpad. On day 21 post vaccination animals were challenged with 1x10⁶ pfu (plaque forming units) LCMV Arm clone13 i.v. (intra venous). 8 days after virus challenge organ virus titer was determined. Points represent individual animals. Dashed line represent detection limit of the assay.

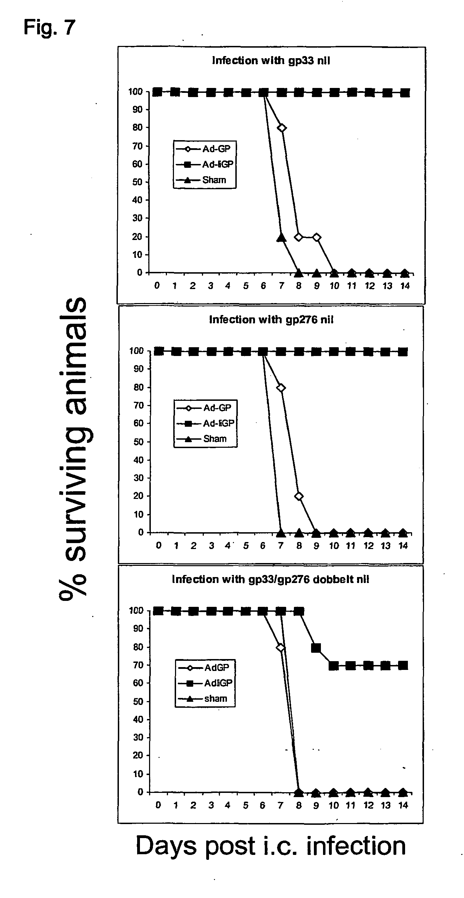

**Figure 7****:** Ad-liGP confers superior protection to lethal LCMV variants with mutations in immunodominant epitopes. C57BL/6 mice were vaccinated with 2x10⁷ IFU of Ad-GP, Ad-liGP or sham infected in the right hind footpad. On day 90 post vaccination animals were challenged with 20 pfu LCMV Arm 53b i.c. carrying mutations in gp33, gp276 or both epitopes. Mortality was recorded for 14 days. For gp33 nil and gp276 nil, each group consisted of 5 animals, with gp33/gp276 double nil, the groups were 10 animals.

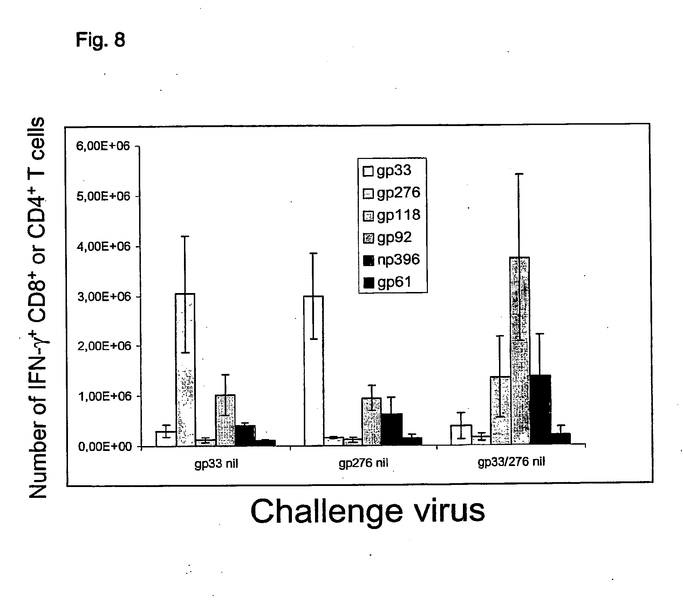

**Figure 8****:** Frequencies of CD8⁺ or CD4⁺ T cells reacting to specific LCMV epitopes after Ad-liGP vaccination and challenge with LCMV variants with mutations in immunodominant epitopes. Surviving animals from the experiment depicted in figure 7 were analysed for epitope specific CD8⁺ or CD4⁺ T cells by intracellular staining for peptide-induced IFN-γ of spleen-cells. Bars represent Avg ± SD of 3-5 animals.

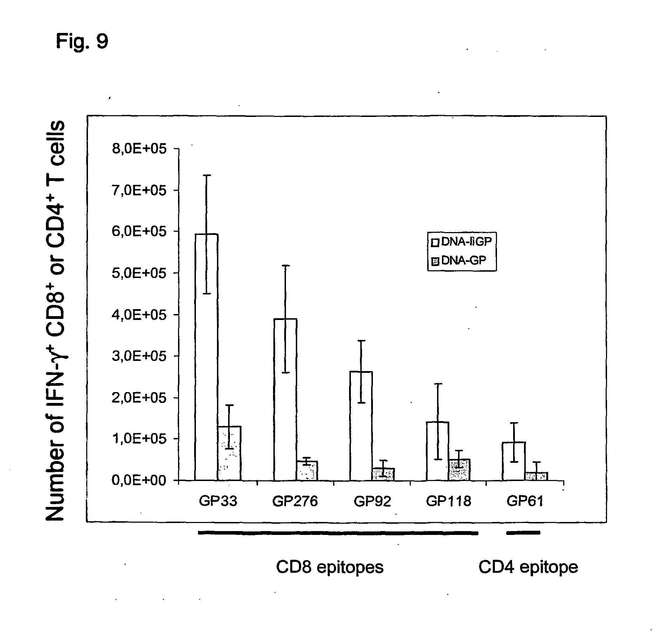

**Figure 9****:** CD8⁺ and CD4⁺ T cell responses to vaccination with naked DNA-liGP and DNA-GP. C57BL/6 mice were vaccinated with DNA coated onto 1.6-nm gold particles in a concentration of 2 µg DNA/mg gold, and the DNA-gold complex was coated onto plastic tubes such that 0.5 mg gold was delivered to the mouse per shot (1 µg DNA per shot). Mice were immunized at the abdominal skin using a hand-held gene gun device employing compressed helium (400 psi) as the particle motive force. Mice were inoculated four times with an interval of 1 week and then allowed to rest for 1 week before investigation. The number of epitope specific CD8⁺ or CD4⁺ T cells was determined by intracellular staining for peptide-induced IFN-γ of spleen cells. Bars represent Avg ± SD of 4-5 animals.

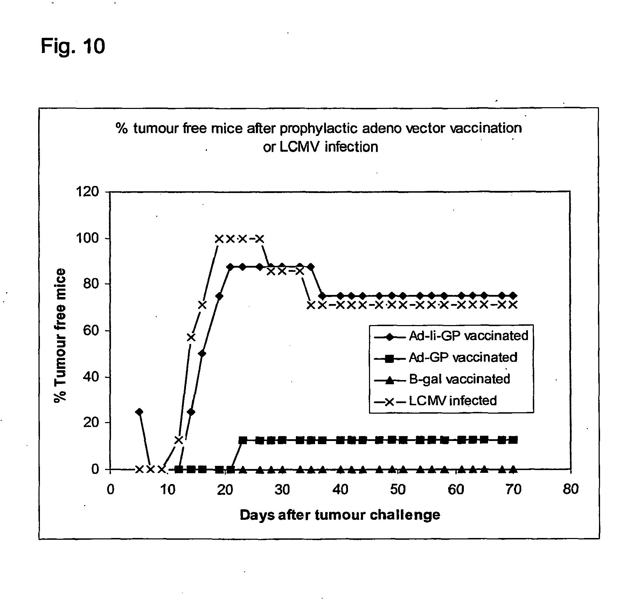

**Figure 10****:** Prophylactic vaccination with Ad-li-GP increases tumor rejection. C57BU6 mice were vaccinated in the right hind foot-pad with 2 x 10⁷ IFU of adenovirus encoding either full-length glycoprotein of LCMV (Ad-GP) or glycoprotein linked to invariant chain (Ad-li-GP). Controls were infected with either adenovirus encoding full-length β-galactosidase or live LCMV (10³ PFU of LCMV Armstrong 53b). About 3 months later all mice were challenged by subcutaneous injection of 10⁶ B16.F10 melanoma cells expressing the LCMV derived GP33 epitope. Initially a tumor was formed in all animals, but the majority of Ad-li-GP and LCMV primed mice eventually rejected the tumor. Each group consisted of 7-10 animals.

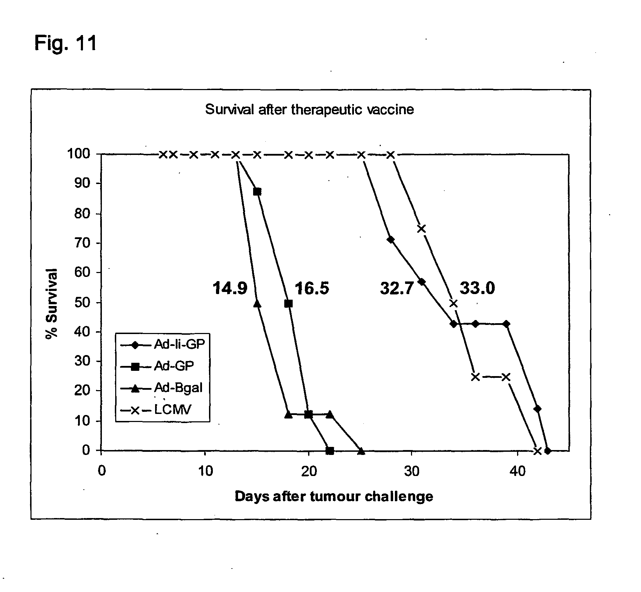

**Figure 11****:** Therapeutic vaccination with Ad-li-GP increases average life span in tumor carrying mice. C57BU6 mice were challenged subcutaneously by injection of 10⁶ B16.F10 melanoma cells expressing the LCMV derived GP33 epitope. When tumors were palpable in all mice (day 5 after tumor injection), the animals were vaccinated in the right hind foot-pad using 2x10⁷ IFU of adenovirus encoding either full-length glycoprotein of LCMV (Ad-GP) or full-length glycoprotein of LCMV linked to invariant chain (Ad-li-GP); controls were vaccinated with either adenovirus encoding full-length β-galactosidase or live LCMV (10³ PFU of LCMV Armstrong 53b). Mice were sacrificed when the tumor exceeded 12 mm in length or ulceration was observed. The numbers in bold in the center of the figure represents the mean day of death following the tumor challenge. Each group consisted of 7-10 animals.

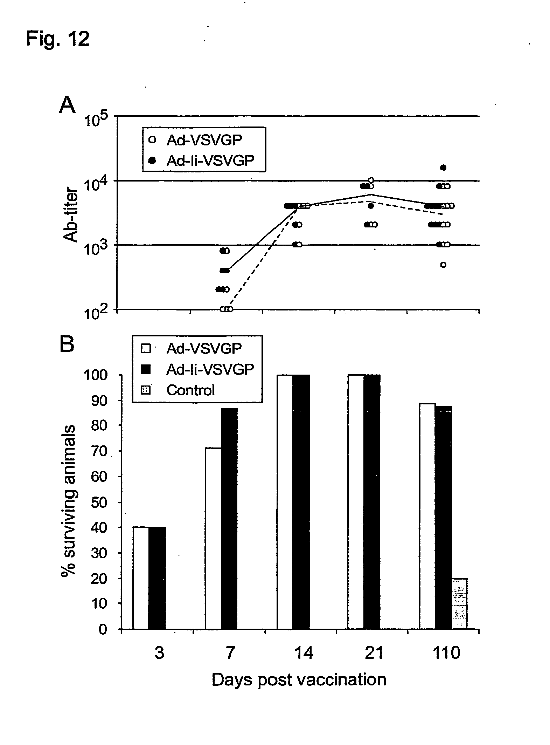

**Figure 12****:** Survival rate following vaccination with either Ad-li-VSVGP or Ad-VSVGP. C57BL/6 mice were vaccinated in the right hind foot-pad with 2x10⁷ IFU of adenovirus encoding either full-length glycoprotein of vesicular stomatitis virus (Ad-VSVGP) or full-length glycoprotein of vesicular stomatitis virus linked to invariant chain (Ad-li-VSVGP). (A) On the indicated days serum samples were collected and *in vitro* neutralizing antibody titers were determined in a plaque-reduction assay, dots represent individual animals. (B) On the indicated days vaccinated mice were challenged with 10⁵ PFU of VSV intranasally, and mortality was registered over the next 14 days. Survival of control (unvaccinated) mice has been included for comparison. Each group consisted of 5-10 animals

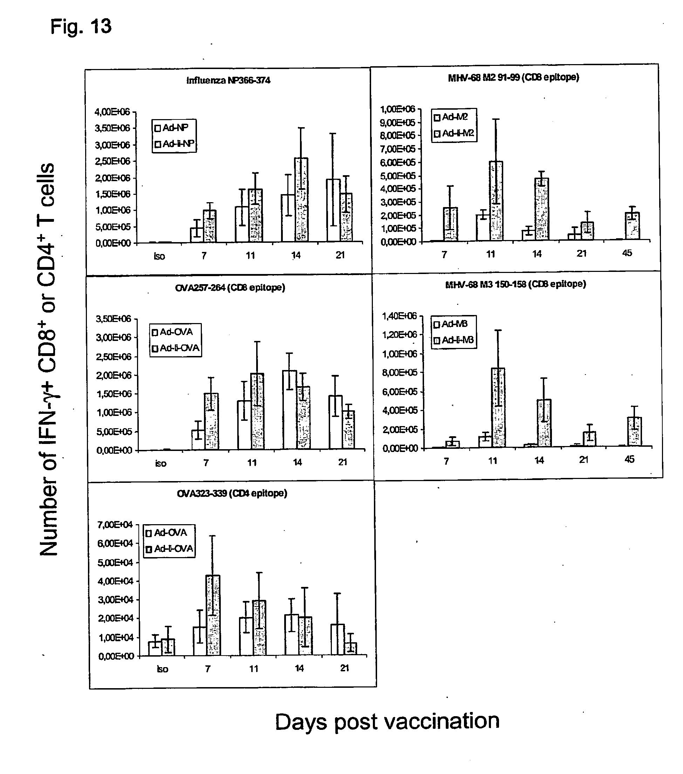

**Figure 13****:** CD8⁺ and CD4⁺ T-cell responses to more adenovirus encoded epitopes. C57BU6 mice (Influenza and OVA) or B6D2 F₁ mice (MHV-68 M2 and M3) were vaccinated with 2x10⁷ IFU of the indicated construct in the right hind footpad. On the indicated days the number of epitope specific CD8+ or CD4+ T cells was determined by intracellular staining for peptide-induced IFN-γ of spleen cells. Iso is the isotype control which determined the background. Bars represent Avg ± SD of 4-5 animals.

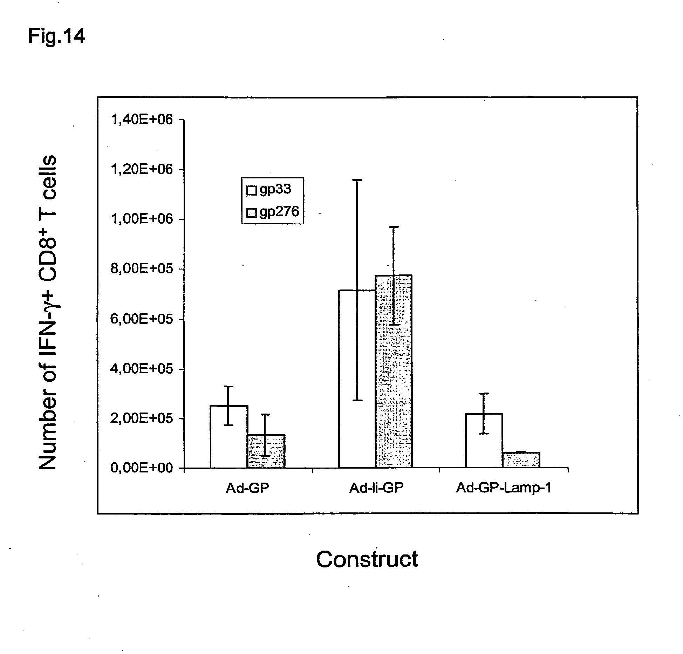

**Figure 14**: Efficiency of Ad-li-GP constructs compared to Ad-GP-Lamp-1 constructs measured by CD8⁺ T-cell responses to adenovirus encoded epitopes. C57BU6 mice were vaccinated with 2x10⁷ IFU of the indicated construct in the right hind footpad. On day 21 the number of epitope specific CD8+ cells was determined by intracellular staining for peptide-induced IFN-γ of spleen cells. Bars represent Avg ± SD of 3 animals.

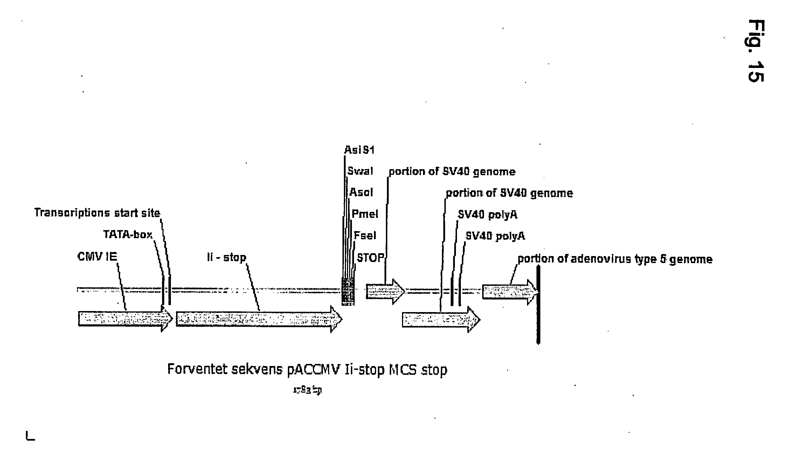

**Figure 15****:** Vectors based on in-frame polylinkers. li without stop-codon was amplified by PCR with the sequence for the AsiSl, Swal, Ascl, Pmel, Fsel and a stop site included in the 3' primer and cloned into the pacCMV vector. The resulting vector was numbered 770 (see partial sequence hereof in SEQ ID NO: 7). A corresponding vector termed 768 (see partial sequence hereof in SEQ ID NO: 8) without the li sequence incorporated was also constructed.

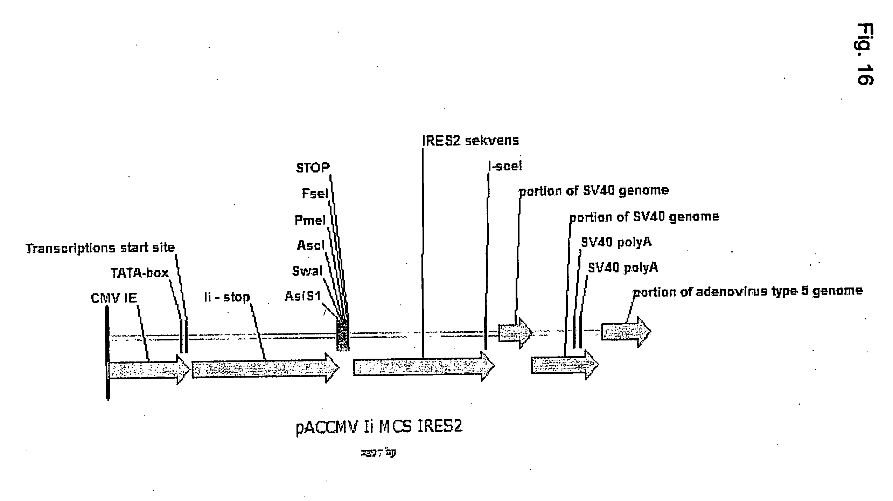

**Figure 16****:** Vectors with IRES2 sites. The vector termed "pacCMV li MCS IRES2" (and numbered 1163, see partial sequence hereof in SEQ ID NO: 9) was constructed by cloning the IRES2 from pLP-IRES2-EGFP into the 770 vector by PCR and restriction enzyme digestion. The sequences for I-scel and Srfl were included in the 3' primer. The first ATG site after the IRES sequence initiates expression of a second protein. A corresponding vector termed "pacCMV MCS IRES2⁻ and numbered 1165 (see partial sequence hereof in SEQ ID NO: 10) without the li sequence incorporated was also constructed

### Examples

The invention will now be further illustrated with reference to the following examples. It will be appreciated that what follows is by way of example only

### Example 1:

CD8⁺ and CD4⁺ T-cell responses to adenovirus encoded epitopes.

*Mice:* C57BU6 (H-2b), C57BU6 x BALB/c (H-2bxd) F1 hybrids and MHC II-/- mice (B6.129-H2-Ab1^{tm1Glm}N12 (H-2b)) were obtained from Taconic M&B (Ry, Denmark). All mice used were between 7-10 weeks old and housed in a specific germ free facility. All experimental procedures were performed according to local experimental guidelines.

*Adenovirus vectors:* For construction of E1 and E3 deleted adenovirus-expressing, LCMV derived antigen fused to invariant chain we performed 2-step PCR. First we obtained overlapping PCR products containing the full-length mouse invariant chain and full-length LCMV glycoprotein and these were joined by secondary PCR with invariant chain 5' and glycoprotein 3' primers. Adenovirus expressing full-length GP was amplified in single step PCR. The obtained fragments were cloned into the pacCMV shuttle vector. The obtained plasmid was co-transfected with pJM17 plasmid into HEK293 cells and viral lysates were obtained. These were cloned by plaque purification before sequencing, large-scale production and purification by CsCl gradient centrifugation as described (Becker et al., 1994, Methods Cell Biol. 43 Pt A:161-189). Infectivity of adenovirus stocks was determined with the Adeno-X Rapid Titer Kit (Clontech). All unmodified virus stocks had particle/IFU ratios between 46 and 201.

*Vaccinations:* In all studies, mice to be vaccinated were anaesthetized and infected with 2x10⁷ infectious units in the right hind footpad in a volume of 0.03 ml.

*Virus infection:* Mice were infected i.c. with 20 pfu of LCMV Armstrong clone 53b in a volume of 0.03 ml or i.v. with 10⁶ pfu of LCMV clone 13 in 0.3 ml. I.c. infection induces a fatal CD8⁺ T cell-mediated meningitis from which immunocompetent mice succumb on days 7 to 10 p.i. (post infection) (Christensen et al., Scandinavian Journal of Immunology 40:373-382).

*Survival study:* Mortality was used to evaluate the clinical severity of acute LCMV-induced meningitis. Mice were checked twice daily for a minimum of 2 weeks after i.c. inoculation; deaths occurring less than 3 days after infection were excluded from analysis.

*Organ virus titers:* To determine virus titers in organs, these were first homogenized in PBS to yield 10% (v/w) organ suspensions, and serial 10-fold dilutions were prepared. Each dilution was then plated in duplicates on MC57G cells. Forty-eight hours after infection, infected cell clusters were detected using monoclonal rat anti-LCMV (VL-4) antibody, peroxidase-labeled goat anti-rat antibody and o-phenylendiamin (substrate) (Battegay et al., 1991, Journal of Virological Methods 33:191-198). The numbers of pfu were counted, and results expressed as pfu/g tissue.

*Cell preparations:* Single cell suspensions of spleen cells were obtained by pressing the organs through a fine steel mesh.

*Abs for flow cytometric:* The following monoclonal antibodies (mAbs) were purchased from BD PharMingen (San Diego, CA) as rat anti-mouse antibody: Cychrome-conjugated anti-CD8, FITC-conjugated anti-CD44, phycoerythrin (PE)-conjugated anti-IFN-γ and PE-conjugated IgG₁ isotype standard.

*Flow cytometri analysis:* Staining of cells for flow cytometry was performed according to standard laboratory procedure (Andersson et al., 1994, Journal of Immunology 152:1237-1245; Andreasen et al., 1999, International Immunology 11:1463-1473). For enumeration of LCMV-specific CD8⁺ T cells, splenocytes were incubated in vitro for 5 h at 37 °C in 5% CO₂ with relevant peptide (0.1 µg/ml) in the presence of monensin (3 µM, Sigma Chemicals co., St. Louis, MO) and murine recombinant IL-2 (10 units/well, R&D Systems Europe Ltd, Abingdon, UK). After incubation cells were surface stained, washed, fixed and permeabilized using 0.5% saponin. Cells were then stained with anti-IFN-γ or IgG₁ isotype control for 20 min at 4 °C. Samples were analyzed using a Becton Dickinson FACSCalibur, and at least 10⁴ mononuclear cells were gated using a combination of low angle and side scatter to exclude dead cells and debris. Data analysis was conducted using Cell Quest Pro (B&D Biosciences).

Replication deficient adenovirus vectors expressing lymphocytic choriomeningitis virus full-length glycoprotein (Ad-GP), or lymphocytic choriomeningitis virus full-length glycoprotein N-terminally linked to murine invariant chain ((Ad-liGP) for schematic representation of the expression cassette see figure 1), were generated through standard methods (Becker et al., Methods Cell Biol. 43 Pt A:161-189). C57BU6 mice were then vaccinated in the right hind paw with 2x10⁷ infectious units of Ad-GP or Ad-liGP and mice were sacrificed 5, 7, 11, 14, 21, 28, 90, 180 or 360 days later. The generation of LCMV glycoprotein specific T-cells were then analysed on splenic cells. Evidently (see figure 2), at all time points tested, Ad-liGP induced numerically superior T-cell responses compared to Ad-GP, and these were accelerated and included both CD4⁺ and CD8⁺ T-cell responses. Thus peak numbers of T-cells generated after Ad-liGP vaccination were obtained at 7-14 days after vaccination depending on the epitope, with responses after Ad-GP peaking at day 21 after vaccination.

### Example 2:

CD8⁺ and CD4⁺ T-cell responses to adenovirus encoded epitopes in F₁ hybrid mice: As C57BU6 mice are homozygous with regard to both MHC class I and MHC class II molecules on all loci, we tested whether Ad-GP and Ad-liGP could also induce an immune response in C57BU6 x BALB/c F₁ mice that express both the H-2^{b} arid H2^{d} haplotypes. These mice resemble an out bred population, but with defined haplotypes. The experiments were performed as above, but testing was limited to day 21 after vaccination. As can be seen from figure 3, Ad-liGP efficiently induces CD8⁺ T-cell responses towards a multitude of epitopes while Ad-GP seemed to perform worse than in homozygous C57BU6 mice.

### Example 3:

Ad-liGP exerts CD8⁺ T-cell stimulatory effects that are independent of CD4⁺ T-cells: As a potential mechanism of li function in the enhanced stimulation of CD8⁺ T-cells is the ability to traffic to endosomal and lysosomal compartments and stimulate CD4⁺ T-cells (Diebold et al., 2001, Gene Ther. 8:487-493) through MHC class II, we performed vaccination of MHC class II deficient mice. To this effect MHC-II^{-/-} or C57BU6 mice were vaccinated with 2x10⁷ IFU of Ad-GP or Ad-liGP in the right hind footpad. On day 21 or 90 post vaccination the number of epitope specific CD8⁺ or CD4⁺ T cells were determined by intracellular staining for peptide-induced IFN-γ of spleen cells. As can be seen from figure 4, Ad-liGP efficiently induces CD8⁺ T-cell responses directed against several epitopes; in the absence of CD4⁺ T cell help however, some responses were lower than what is seen in wild type mice.

### Example 4:

Ad-liGP confers rapid, superior and sustained protection against lethal LCMV infection: As we observed an accelerated response to Ad-liGP compared to Ad-GP we investigated the ability of vaccination to confer protection both at 21 days post vaccination (peak of Ad-GP) and at 3, 5, 7, 14, 60, 90, 180 and 360 days post vaccination (Figure 5). Remarkably, we found that Ad-liGP vaccinated animals vaccinated as little as 3 days previously were protected against intracerebral LCMV infection. Protection conferred by Ad-GP was only partial at 14 and 21 days post infection and no protection were seen at day 60 or later. Furthermore, the Ad-liGP conferred protection was sustained for 360 days, at which point Ad-GP no longer protected against intracerebral LCMV infection.

### Example 5:

Ad-liGP efficiently protects against high-dose, intravenous LCMV infection: Since we found that Ad-liGP protected mice against an acute localised infection, we wanted to investigate whether the same held true for a high-dose systemic infection. Accordingly, mice were vaccinated with Ad-GP, Ad-liGP or sham (PBS), and challenged 21 days later by intravenous injection of 10⁶ plaque-forming units of the fast replicating LCMV clone 13 strain. 5 days later animals were sacrificed and infectious titers in the lungs were determined. Although Ad-GP conferred significant protection upon vaccinated animals, Ad-liGP was superior and reduced titers to a level at or below the detection limit of the assay (Figure 6).

### Example 6:

Comparative analyses of novel constructs with respect to kinetics, magnitude of response, long-term immunity and virus dose needed for immunity.

All constructs described below are comparatively analysed among each other and to Ad-GP and Ad-liGP with respect to the kinetics and magnitude of immune response e.g. as described in Examples 1, 2 and 3, long-term immunity e.g. as described in Examples 1, 4 and 5, and virus dose needed for immunity.

Novel constructs based upon alterations to the li fusions. In each construct adenovirus-encoded GP is fused N-terminally to:
1. the lysosomal targeting sequence of li
2. the CLIP sequence of li
3. the KEY sequence of li
4. the CLIP sequence and the sequence N-terminally adjacent to the CLIP sequence
5. the CLIP sequence and the sequence C-terminally adjacent to the CLIP sequence
6. the sequence N-terminally adjacent to the CLIP sequence
7. the sequence C-terminally adjacent to the CLIP sequence

Alterations of antigen presentation context. In each construct adenovirus-encoded GP is fused:
1. C-terminally to LAMP
2. N-terminally to the N-domain of calreticulin
3. C-terminally to the N-domain of calreticulin
4. C-terminally to Hsp70.
5. N-terminally to li and C-terminally to the N-domain of calreticulin (AdliGPCrt)
6. N-terminally to li and C-terminally to Hsp70 (AdliGPHsp70)

All of these constructs are furthermore used as the starting point for a series of constructs in which the GP-fusions are followed by an internal ribosomal entry site (IRES) and a gene encoding VP22, HIV tat or Cx43.

Alterations with regard to intercellular spreading. In each construct adenovirus-encoded GP is fused:
1. N-terminally to herpes simplex virus encoded VP22
2. N-terminally to HIV encoded tat
3. N-terminally to connexin 43 (Cx43)
4. N-terminally to other connexins and intercellular gap-junctions constituents.

Furthermore constructs are prepared where adenovirus-encoded GP is followed by an internal ribosomal entry site (IRES) and a gene encoding VP22, HIV tat or Cx43 or other connexins and intercellular gap-junctions constituents.

All of the above constructs are furthermore altered in any of the following ways:
1. All of the above constructs followed by an IRES site and a gene encoding an NK-cell (natural killer cell) activation molecule, for example H60. This alteration gives enhanced delivery of co-stimulatory signals and cytokine help.
2. All of the above constructs involving IRES sites, where the downstream gene is placed under control of a separate promoter.
3. All of the above constructs involving IRES sites, where the downstream gene is instead encoded on a separate vector.
4. All of the above constructs placed under inducible promoter systems.
5. All of the above constructs placed under cell type specific and/or inducible promoter systems.
6. All of the above constructs where the GP antigenic sequence is replaced with a sequence encoding any of the following antigens, several of which comprise multiple antigens, see examples of specific antigens in figures 12 and 13: VSV-GP, Influenza A NS-1, Influenza A M1, Influenza A NP, LCMV NP, LCMV GP, Ebola GP Ebola NP, murine gammaherpesvirus (MHV-68) M2, M3 (this corresponds to the human EBV and HHV8 viruses) and ORF73, chicken Ovalbumin (OVA), or a helper T-cell epitope. These antigenic sequences will furthermore be combined so at least 2 or more are encoded in the same vector.

See figure 14 for an example of a comparison of the efficiency of Ad-li-GP pg33 and gp276 constructs compared to Ad-GP-Lamp-1 pg33 and gp276 constructs as measured by CD8+ T-cell responses. As can be seen, the Ad-li-GP constructs are superior to the Ad-GP-Lamp-1 constructs in their capability of evoking a CD8+ T-cell response.

### Example 7:

Comparative analyses of novel constructs and alternative administration methods with respect to kinetics, magnitude of response, long-term immunity and virus dose needed for immunity.

All constructs described in Example 6 are comparatively analysed among each other and to Ad-GP and Ad-liGP following alternative administration methods. The comparisons are done with respect to the kinetics and magnitude of immune response e.g. as described in Examples 1, 2 and 3, long-term immunity e.g. as described in Examples 1, 4 and 5, and virus dose needed for immunity.

Alternative administration methods include:
1. Alterations with regard to enhanced delivery of co-stimulatory signals and cytokine help in which all of the in Example 6 described constructs are co-injected with adenovirus encoded type 1 interferon, for example tetracycline inducible IFN-β. Furthermore, all the in Example 6 described constructs are co-injected with adenovirus encoded cytokine, for example IL-15.
2. Administration of Ad-liGP simultaneously with Ad-Tet-onGP at separate sites of the body (Ad-Tet-onGP encodes GP under control of a tetracycline inducible promoter).
3. Adenovirally delivery of any one of the in Example 6 described inserts/constructs followed by homologous viral vector boosting with the same insert/construct or followed by heterologous viral vector boosting with lentivirus encoded or other adenovirus-encoded delivery of the same insert/construct.

### Example 8:

Ad-liGP confers rapid and superior protection against lethal LCMV infection in absence of major epitopes. Lymphocytic choriomeningitis virus full-length glycoprotein (GP) comprises four CD8⁺ specific epitopes of varying antigenicity, measured by the percentile of CD8⁺ cells with specificity for the individual epitope against all the CD8⁺ cells raised in response to GP vaccination. The predominant population of CD8⁺ cells raised against GP is specific for the gp33 epitope, a somewhat smaller population is specific for gp276, and minor populations are specific for gp118 and for gp92. As gp33 and gp276 are the major / immunodominant epitopes, we investigated how nil mutations of either epitope independently or both simultaneously would effect the efficiency of the protection offered by the Ad-liGP fusion construct.

C57BU6 mice were vaccinated with 2x10⁷ IFU Ad-GP and Ad-liGP or sham infected in the right hind footpad. Each group consisted of 5 animals. On day 90 post vaccination, the animals were challenged with 20 pfu LCMV Arm 53b i.c. (intra cerebral) constructs carrying gp33 nil mutations, gp276 nil mutations or gp33/gp276 double nil mutations. Mortality was recorded for 14 days. As can be seen from figure 7, Ad-liGP conferred superior protection against lethal LCMV infection despite the gp33 or gp276 nil mutations. The double nil mutation of gp33/gp276 lead to the survival of 70% of the animals compared to no surviving animals in the Ad-GP and Sham vaccinated groups.

Surviving animals from the experiment above were analyzed for epitope specific CD8⁺ or CD4⁺T cells by intracellular staining for peptide-induced IFN-γ of spleen cells. Expectedly, as can be seen from figure 8, in the absence of either gp33 or gp276, the major epitope specificity of the CD8⁺ or CD4⁺T cells is gp276 or gp33, respectively. In the absence of both gp33/gp276, the major epitope specificity of the CD8⁺ or CD4⁺T cells is gp92.

### Example 9:

CD8⁺ and CD4⁺ T cell responses to vaccination with naked DNA-liGP and DNA-GP. To investigate an alternative platform for invariant chain fusion vaccines other than adenoviral delivery, we tested the ability of liGP and GP as naked DNA to raise GP-epitope specific CD8⁺ and CD4⁺ T cells. The naked DNA comprised the GP and li-GP fragments from the Adenoviral vectors illustrated in figure 1.

C57BU6 mice were vaccinated with DNA coated onto 1.6-nm gold particles in a concentration of 2 µg DNA/mg gold, and the DNA-gold complex was coated onto plastic tubes such that 0.5 mg gold (1 µg DNA per shot) was delivered to the mouse per shot. Mice were immunized at the abdominal skin using a hand-held gene gun device employing compressed helium (400 psi) as the particle motive force. Mice were inoculated four times with an interval of 1 week and then allowed to rest for 1 week before investigation. The number of epitope specific CD8⁺ or CD4⁺ T cells were determined by intracellular staining for peptide-induced IFN-γ of spleen cells. As can be seen from figure 9, DNA-liGP efficiently induces CD8⁺ T-cell responses directed against several epitopes.

### Example 10:

Ad-liGP confers superior protection against challenge with tumor cells expressing the gp33 epitope from LCMV. Under normal circumstances, tumor cells express several different antigens recognized by T cells. To determine the efficiency of this response, B16.F10 melanoma cells expressing gp33 from LCMV were used to challenge vaccinated animals with.

C57BU6 mice were vaccinated with 2x10⁷ IFU Ad-GP or Ad-liGP in the right hind footpad. As a control some animals were vaccinated with Ad-β-galactosidase (negative control) or infected with LCMV (positive control). On day 90 after vaccination/infection animals were challenged with 10⁶ tumor cells subcutaneously and the tumor growth was followed by measuring the size of the tumor. Initially a tumor will form in all animals, but eventually the immune response directed towards gp33 will eliminate the tumor cells. Each group consisted of 7-10 animals. Prophylactic vaccination with Ad-li-GP resulted in tumor free mice in 70% of the cases compared to only 10% in Ad-GP vaccinated animals, as can be seen from figure 10.

In many cases the tumor has already formed when a physician sees the patient. To mimic this situation, C57BU6 mice were injected with 10⁶ B16.F10 tumor cells subcutaneously. After 5 days the tumors were palpably recognizable and could be measured. At this time point animals were vaccinated with 2x10⁷ IFU Ad-GP or Ad-liGP in the right hind footpad. As a control some animals were vaccinated with Ad-β-galactosidase (negative control) or infected with LCMV (positive control). Tumor growth was followed by measuring the size of the tumor, and once the size was greater that 12 mm in any dimension the animals were sacrificed. As can be seen from figure 11, in mice given the therapeutic Ad-liGP vaccine, the speed of tumor development was approximately half of that seen in mice given Ad-GP as therapeutic vaccine, as measured by number of days passing prior to reaching a 12 mm tumor size and animal sacrifice.

### Example 11:

To demonstrate that the vaccine construct also can induce protecting antibody response the VSV infection was used.

*Virus and virus quantitation:* Vesicular stomatitis virus (VSV) of the Indiana serotype was used throughout this study. Stocks of virus were propagated in L929 cells (ATCC CCL 1) and stored at -70 °C until use. Virus quantitation was performed by plaque assay on monolayers of L929 cells. In brief, serial 10-fold dilutions of virus were prepared in Eagle's minimal essential medium (F11) containing 1% L-glutamine, 1 % penicillin/streptomycin, 5% NaHCO₃ and 10% fetal calf serum. One ml of each dilution was added in duplicate to monolayers of L929 cells in petri dishes plated 48 hours earlier. After incubation for 90 min at 37°C in 5% CO₂, medium containing the virus dilutions was aspirated, and the monolayers were overlaid with a mixture of 2.5 ml 1% agarose and 2.5 ml 2 x F11. Monolayers were then incubated for 24 hours at 37°C in 5% CO₂ before staining with a mixture of 1 ml of 1% agarose and 1 ml of 2xF11 containing 1% neutralred. After further 24 hours of incubation, the numbers of PFU were counted.

*Survival study:* Mortality was used as parameter for the severity of VSV infection, based on previous findings that virus titer in CNS correlate strongly with clinical symptoms (Thomsen et al. 1997, Int. Immunol. 9:1757-1766.). Mice were inspected daily for signs of VSV-induced paralysis, and sacrificed when severe paralysis was noted and the animals expected to succumb within the next 24 hours.

*Serum neutralizations test:* Serial two-fold dilutions of serum in F11 were mixed with equal volumes of virus diluted to contain approximately 100 PFU/ml. After 1 h of incubation at room temperature, 1 ml of each serum-virus mixture was added in duplicate to monolayers of L929 cells in petri dishes and assayed for the presence of residual virus by plaque assay (see virus and virus quantitation). The highest serum dilution that reduced the number of plaques by at least 50% was taken as the neutralizing titer.

C57BU6 mice were vaccinated in the right hind foot-pad with 2x10⁷ IFU of adenovirus encoding either full-length glycoprotein of vesicular stomatitis virus (Ad-VSVGP) or glycoprotein linked to invariant chain (Ad-li-VSVGP). On day 7, 14, 21 and 110 after vaccination serum samples were collected and in vitro neutralizing antibody titers were determined in a plaque-reduction assay, figure 12a. On day 3, 7, 14, 21 and 110 after vaccination, animals were challenged with 10⁵ PFU of VSV intranasally, and mortality was registered over the next 14 days, figure 12b. As seen in figure 12 almost identical responses are seen in the two groups, suggesting that, although not an advantage, the invariant chain allows for antibody production and does not have a detrimental effect on the production.

### Reference List

Andersson, E. C., J. P. Christensen, O. Marker and A. R. Thomsen. 1994. Journal of Immunology 152:1237-1245. Changes in Cell Adhesion Molecule Expression on T Cells Associated with Systemic Virus Infection.
Andreasen, S. Ø., J. P. Christensen, O. Marker and A. R. Thomsen. 1999. International Immunology 11:1463-1473. Virus induced non-specific signals cause cell cycle progression of primed CD8+ T cells but do not induce cell differentiation.
Battegay M., S. Cooper, A. Althage, J. Bänziger, H. Hengartner, and R.M. Zinckemagel.1991. Journal of Virological Methods 33:191-198. Quantification of lymphocytic choriomenigitis virus with an immunological focus assay in 24- or 96-well plates.
Becker, T. C., R. J. Noel, W. S. Coats, A. M. Gomez-Foix, T. Alam, R. D. Gerard, and C. B. Newgard. 1994. Use of recombinant adenovirus for metabolic engineering
Christensen, J. P., O. Marker, A. R. Thomsen. 1994. Scandinavian Journal of Immunology 40:373-382. The role of CD4+ T cells in cell-mediated immunity to LCMV: studies in MHC class I and class II deficient mice.
Diebold, S. S., M. Cotten, N. Koch, and M. Zenke. 2001. MHC class II presentation of endogenously expressed antigens by transfected dendritic cells. Gene Ther. 8:487-493.
Morris, C. R., A. J. Reber, J. L. Petersen, S. E. Vargas, and J. C. Solheim. 2004. Association of intracellular proteins with folded major histocompatibility complex class I molecules. Immunol.Res. 30:171-179.
Pieters, J. 1997. MHC class II restricted antigen presentation. Curr.Opin.Immuno/. 9:89-96.
Sambrook et al., eds., Molecular Cloning: A Laboratory Manual, Cold Spring Harbor Laboratory, 2nd Edition, Cold Spring Harbor, N.Y., 1989.
Strumptner-Cuvelette, P., and P. Benaroch. 2002. Biochem. Biophys. Acta., 1542:1-13. Multiple roles of the invariant chain in MHC class II function.
Thomsen, R. A., A. Nansen, C. Andersen, J. Johansen, O. Marker, and J. P. Christensen. 1997. International Immunology 9:1757-1766. Cooperation of B cells and T cells is required for survival of mice infected with vesicular stomatitis virus.

## Claims

1. An adenoviral vector comprising a nucleotide construct encoding:
a) at least one antigen and
b) at least one protein or peptide or fragment of a protein or peptide which stimulates an MHC-I response, wherein the at least one MHC-I response stimulating protein or peptide or fragment of protein or peptide is invariant chain having at least 85% identity to SEQ ID NO: 2 or a fragment of the sequence identified in SEQ ID NO:2 of at least 40 amino acids and of at least 85% identity to the same fragment of SEQ ID NO: 2, wherein the invariant chain or the fragment comprises a CLIP region.

2. The adenoviral vector according to claim 1, wherein at least one signal peptide is added to, removed from or replaces the signal peptide of the at least one invariant chain.

3. The adenoviral vector according any of the preceding claims, wherein at least one antigenic protein or peptide or an antigenic fragment of said protein or peptide is selected from the group of: pathogenic organisms, cancer-specific polypeptides, and proteins or peptides associated with an abnormal physiological response or is an antigenic protein or peptide or an antigenic fragment of said protein or peptide at least 85% identical to an antigen from the above group.

4. The adenoviral vector according to any of the preceding claims, wherein the at least one antigenic protein or peptide or an antigenic fragment of said protein or peptide from a pathogenic organism is selected from the group of pathogens comprising: virus, micro organisms and parasites,
wherein the virus is preferably selected from the group consisting of: HIV, Hepatitis C virus, influenza virus, herpes virus, Lassa, Ebola, smallpox, Bird flu, filovirus, Marburg, papillomavirus or,
wherein the micro organism preferably is selected from the group consisting of: Mycobacterium tuberculosis, Bacillus anthracis, Staphylococcus species, and Vibrio species, or
wherein the parasite preferably is selected from the group consisting of: Plasmodium species, Leishmania species, and Trypanosoma species.

5. The adenoviral vector according to any of the preceding claims, wherein at least one antigenic protein or peptide or an antigenic fragment of said protein or peptide is from a cancer-specific polypeptide.

6. The adenoviral vector according to any of the preceding claims, wherein at least one antigenic protein or peptide or an antigenic fragment of said protein or peptide is from a polypeptide associated with an abnormal physiological response, wherein the abnormal physiological response is an autoimmune disease, an allergic reaction, cancer or a congenital disease.

7. The adenoviral vector according to any of the preceding claims, wherein the operative link between the invariant chain or fragment and the antigenic protein or peptide or an antigenic fragment of said protein or peptide is selected from the group of: a direct link or a link mediated by a spacer region.

8. The adenoviral vector according to claim 7, wherein the operative linker is a spacer region, wherein the spacer region encodes at least one helper epitope for class II MHC molecules or wherein the spacer region encodes at least one protease cleavage site.

9. The adenoviral vector according to any of the preceding claims, wherein at least one invariant chain or fragment is operatively linked to at least two, antigenic proteins or peptides or an antigenic fragment of said protein or peptide.

10. The adenoviral vector according to any of the preceding claims, wherein the vector is a replication defective adenovirus or a conditionally replication deficient adenovirus.

11. A vaccine composition comprising an adenoviral vector comprising a nucleic acid sequence encoding:
a) at least one operatively linked protein or peptide or fragment of a protein or peptide which stimulates an MHC-I response, wherein the at least one MHC-I response stimulating protein or peptide or fragment of protein or peptide is invariant chain having at least 85% identity to SEQ ID NO:2 or a fragment of the sequence identified in SEQ ID NO:2 of at least 40 amino acids and of at least 85% identity to the same fragment of SEQ ID NO: 2, wherein the invariant chain or the fragment comprises a CLIP region operatively linked to
b) at least one antigenic protein or peptide or an antigenic fragment of said protein or peptide
for use as a medicament.

12. The vaccine according to claim 11, comprising an adenoviral vector as defined in any of claims 1 to 10.

13. The vaccine according to claims 11 or 12, wherein the vaccine comprises means for intramuscular, intravenous or subcutaneous administration.

14. The vaccine composition according to any of claims 11 to 13, comprising a second active ingredient wherein the second active ingredient is selected from the group of: antibiotics, chemotherapeutics, anti-allergenics, cytokines and co-stimulatory molecules of the immune system.

15. A kit of parts comprising:
- a vaccine composition comprising an adenoviral vector according to any of the claims 11 to 14,
- a medical instrument or other means of administering said vaccine,
- instructions on how to use the kit in parts.

16. Use of the adenoviral vector according to any of claims 1 to 10, for the production of a vaccine.

## Patentansprüche

1. Adenoviraler Vektor, der ein Nukleotidkonstrukt umfasst, welches kodiert:
a) mindestens ein Antigen und
b) mindestens ein Protein oder Peptid oder Fragment eines Proteins oder Peptids, das eine MHC-I-Antwort stimuliert, wobei das mindestens eine eine MHC-I-Antwort stimulierende Protein oder Peptid oder Fragment eines Proteins oder Peptids eine invariante Kette ist, die mindestens 85% Identität zu SEQ ID NO: 2 oder zu einem Fragment der durch SEQ ID NO: 2 identifizierten Sequenz von wenigstens 40 Aminosäuren und mit wenigstens 85% Identität zu demselben Fragment von SEQ ID NO: 2 aufweist, wobei die invariante Kette oder das Fragment eine CLIP-Region umfasst.

2. Adenoviraler Vektor gemäß Anspruch 1, wobei mindestens ein Signalpeptid zu dem Signalpeptid der mindestens einen invarianten Kette hinzugefügt wird, aus diesem entfernt wird oder dieses ersetzt.

3. Adenoviraler Vektor gemäß einem der vorangehenden Ansprüche, wobei mindestens ein antigenes Protein oder Peptid oder ein antigenes Fragment des Proteins oder Peptids aus der Gruppe ausgewählt ist: pathogene Organismen, Krebs-spezifische Polypeptide und Proteine oder Peptide, die mit einer abnormalen physiologischen Antwort assoziiert sind, oder es ist ein antigenes Protein oder Peptid oder ein antigenes Fragment des Proteins oder des Peptids, das mindestens 85% identisch zu einem Antigen aus der obigen Gruppe ist.

4. Adenoviraler Vektor gemäß einem der vorangehenden Ansprüche, wobei das mindestens eine antigene Protein oder Peptid oder ein antigenes Fragment des Proteins oder Peptids aus einem pathogenen Organismus aus der Gruppe von Pathogenen ausgewählt ist, die umfasst: Viren, Mikroorganismen und Parasiten, wobei das Virus vorzugsweise ausgewählt ist aus der Gruppe bestehend aus: HIV, Hepatitis-C-Virus, Influenzavirus, Herpesvirus, Lassavirus, Ebolavirus, Pockenvirus, Vogelgrippevirus, Filovirus, Marburgvirus und Papillomavirus, oder wobei der Mikroorganismus vorzugsweise ausgewählt ist aus der Gruppe bestehend aus: Mycobacterium tuberculosis, Bacillus anthracis, Staphylococcus-Spezies und Vibrio-Spezies, oder
wobei der Parasit vorzugsweise ausgewählt ist aus der Gruppe bestehend aus: Plasmodium-Spezies, Leishmania-Spezies und Trypanosoma-Spezies.

5. Adenoviraler Vektor gemäß einem der vorangehenden Ansprüche, wobei mindestens ein antigenes Protein oder Peptid oder ein antigenes Fragment des Proteins oder Peptids aus einem Krebs-spezifischen Polypeptid stammt.

6. Adenoviraler Vektor gemäß einem der vorangehenden Ansprüche, wobei mindestens ein antigenes Protein oder Peptid oder ein antigenes Fragment des Proteins oder Peptids aus einem Polypeptid stammt, das mit einer abnormalen physiologischen Antwort assoziiert ist, wobei die abnormale physiologische Antwort eine Autoimmunerkrankung, eine allergische Reaktion, Krebs oder eine angeborene Erkrankung ist.

7. Adenoviraler Vektor gemäß einem der vorangehenden Ansprüche, wobei die wirksame Verknüpfung zwischen der invarianten Kette oder dem Fragment und dem antigenen Protein oder Peptid oder einem antigenen Fragment des Proteins oder Peptids ausgewählt ist aus der Gruppe einer direkten Verknüpfung oder einer Verknüpfung, die durch eine Spacer-Region vermittelt wird.

8. Adenoviraler Vektor gemäß Anspruch 7, wobei der wirksame Linker eine Spacer-Region ist, wobei die Spacer-Region mindestens ein Helfer-Epitop für Klasse-II-MHC-Moleküle kodiert oder wobei die Spacer-Region mindestens eine Proteaseschnittstelle kodiert.

9. Adenoviraler Vektor gemäß einem der vorangehenden Ansprüche, wobei mindestens eine invariante Kette oder ein Fragment operativ mit mindestens zwei antigenen Proteinen oder Peptiden oder einem antigenen Fragment des Proteins oder des Peptids verknüpft ist.

10. Adenoviraler Vektor gemäß einem der vorangehenden Ansprüche, wobei der Vektor ein replikationsdefizienter Adenovirus oder ein bedingt replikationsdefizienter Adenovirus ist.

11. Impfstoffzusammensetzung, die einen adenoviralen Vektor umfasst, der eine Nukleinsäuresequenz umfasst, welche kodiert:
a) mindestens ein operativ verbundenes Protein oder Peptid oder ein Fragment eines Proteins oder Peptids, welches eine MHC-I-Antwort stimuliert, wobei das mindestens eine MHC-I-Antwort stimulierende Protein oder Peptid oder Fragment des Proteins oder Peptids eine invariante Kette ist, die wenigstens 85% Identität zu SEQ ID NO: 2 aufweist, oder ein Fragment der durch SEQ ID NO: 2 identifizierten Sequenz von wenigstens 40 Aminosäuren und mit wenigstens 85% Identität zu demselben Fragment von SEQ ID NO: 2 ist, wobei die invariante Kette oder das Fragment eine CLIP-Region umfasst, die operativ verknüpft ist mit
b) mindestens einem antigenen Protein oder Peptid oder einem antigenen Fragment des Proteins oder Peptids
zur Verwendung als ein Medikament.

12. Impfstoff gemäß Anspruch 11 umfassend einen adenoviralen Vektor wie er in einem der Ansprüche 1-10 definiert ist.

13. Impfstoff gemäß Anspruch 11 oder 12, wobei der Impfstoff Mittel zur intramuskulären, intravenösen oder subkutanen Verabreichung umfasst.

14. Impfstoffzusammensetzung gemäß einem der Ansprüche 11-13, die einen zweiten aktiven Inhaltsstoff umfasst, wobei der zweite aktive Inhaltsstoff ausgewählt ist aus der Gruppe: Antibiotika, Chemotherapeutika, Antiallergika, Cytokine und costimulierende Moleküle des Immunsystems.

15. Kit-of-parts, umfassend:
- Impfstoffzusammensetzung, die einen adenoviralen Vektor umfasst, gemäß einem der Ansprüche 11-14,
- ein medizinisches Instrument oder andere Mittel zum Verabreichen des Impfstoffs,
- Anweisungen darüber, wie das Kit-in-parts zu verwenden ist.

16. Verwendung des adenoviralen Vektors gemäß einem der Ansprüche 1-10 zur Herstellung eines Impfstoffs.

## Revendications

1. Vecteur adénoviral comprenant une construction nucléotidique codant pour:
a) au moins un antigène et
b) au moins une protéine ou un peptide ou un fragment d'une protéine ou d'un peptide qui stimule une réponse par CMH-I, tandis que la au moins une protéine ou un peptide ou un fragment de protéine ou de peptide stimulant la réponse par CMH-I est une chaîne invariante ayant au moins 85% d'identité vis-à-vis de SEQ ID NO: 2 ou d'un fragment de la séquence identifiée dans SEQ ID NO: 2 ayant au moins 40 acides aminés et au moins 85% d'identité vis-à-vis du même fragment de SEQ ID NO: 2, tandis que la chaîne invariante ou le fragment comprend une région CLIP.

2. Vecteur adénoviral selon la revendication 1, dans lequel au moins un peptide signal est ajouté au, éliminé du ou remplace le peptide signal de la au moins une chaîne invariante.

3. Vecteur adénoviral selon l'une quelconque des revendications précédentes, dans lequel au moins une protéine ou un peptide antigénique ou un fragment antigénique de ladite protéine ou dudit peptide est choisi dans le groupe des: organismes pathogènes, polypeptides spécifiques d'un cancer, et protéines ou peptides associés à une réponse physiologique anormale ou est une protéine ou un peptide antigénique ou un fragment antigénique de ladite protéine ou dudit peptide ayant au moins 85% d'identité vis-à-vis d'un antigène du groupe susdit.

4. Vecteur adénoviral selon l'une quelconque des revendications précédentes, dans lequel la au moins une protéine ou le au moins un peptide antigénique ou un fragment antigénique de ladite protéine ou dudit peptide provenant d'un organisme pathogène est choisi dans le groupe des agents pathogènes comprenant : virus, micro-organismes et parasites,
tandis que le virus est de préférence choisi dans le groupe consistant en: VIH, virus de l'hépatite C, virus de la grippe, herpèsvirus, virus de Lassa, virus Ebola, virus de la variole, virus de grippe aviaire, filovirus, virus Marburg, et papillomavirus, ou
tandis que le micro-organisme est de préférence choisi dans le groupe consistant en: Mycobacterium tuberculosis, Bacillus anthracis, espèce Staphylococcus, et espèce Vibrio, ou
tandis que le parasite est de préférence choisi dans le groupe consistant en: espèce Plasmodium, espèce Leishmania, et espèce Trypanosoma.

5. Vecteur adénoviral selon l'une quelconque des revendications précédentes, dans lequel au moins une protéine ou un peptide antigénique ou un fragment antigénique de ladite protéine ou dudit peptide provient d'un polypeptide spécifique pour un cancer.

6. Vecteur adénoviral selon l'une quelconque des revendications précédentes, dans lequel au moins une protéine ou un peptide antigénique ou un fragment antigénique de ladite protéine ou dudit peptide provient d'un polypeptide associé à une réponse physiologique anormale, tandis que la réponse physiologique anormale est une affection auto-immune, une réaction allergique, un cancer ou une maladie congénitale.

7. Vecteur adénoviral selon l'une quelconque des revendications précédentes, dans lequel le lien opérationnel entre la chaîne ou le fragment invariant(e) et la protéine ou le peptide antigénique ou un fragment antigénique de ladite protéine ou dudit peptide est choisi dans le groupe de: un lien direct ou un lien avec médiation par une région d'espaceur.

8. Vecteur adénoviral selon la revendication 7, dans lequel le lieur opérationnel est une région d'espaceur, tandis que la région d'espaceur code pour au moins un épitope auxiliaire pour les molécules de CMH de classe II ou tandis que la région d'espaceur code pour au moins un site de clivage par protéase.

9. Vecteur adénoviral selon l'une quelconque des revendications précédentes, dans lequel au moins une chaîne ou un fragment invariant(e) est lié(e) de manière opérationnelle à au moins deux protéines ou peptides antigéniques ou un fragment antigénique de ladite protéine ou dudit peptide.

10. Vecteur adénoviral selon l'une quelconque des revendications précédentes, dans lequel le vecteur est un adénovirus déficient pour la réplication ou un adénovirus conditionnellement déficient pour la réplication.

11. Composition vaccinale comprenant un vecteur adénoviral comprenant une séquence d'acide nucléique codant pour:
a) au moins une protéine ou un peptide ou un fragment d'une protéine ou d'un peptide lié(e) de manière opérationnelle, qui stimule une réponse par CMH-I, tandis que la au moins une protéine ou le au moins un peptide ou fragment de protéine ou de peptide stimulant la réponse par CMH-I est une chaîne invariante ayant au moins 85% d'identité vis-à-vis de SEQ ID NO:2 ou d'un fragment de la séquence identifiée dans SEQ ID NO:2 d'au moins 40 acides aminés et d'au moins 85% d'identité vis-à-vis du même fragment de SEQ ID NO:2, dans lequel la chaîne ou le fragment invariant(e) comprend une région CLIP liée de manière opérationnelle à
b) au moins une protéine ou un peptide antigénique ou un fragment antigénique de ladite protéine ou dudit peptide
pour utilisation comme médicament.

12. Vaccin selon la revendication 11, comprenant un vecteur adénoviral tel que défini selon l'une quelconque des revendications 1 à 10.

13. Vaccin selon les revendications 11 ou 12, dans lequel le vaccin comprend des moyens pour l'administration intramusculaire, intraveineuse ou sous-cutanée.

14. Composition vaccinale selon l'une quelconque des revendications 11 à 13, comprenant un deuxième constituant actif, tandis que le deuxième constituant actif est choisi dans le groupe des: antibiotiques, agents chimiothérapeutiques, agents anti-allergéniques, cytokines et molécules de co-stimulation du système immunitaire.

15. Kit ou ensemble de parties comprenant:
- une composition vaccinale comprenant un vecteur adénoviral selon l'une quelconque des revendications 11 à 14,
- un instrument médical ou d'autres moyens d'administration dudit vaccin,
- des instructions sur la manière d'utiliser le kit de parties.

16. Utilisation du vecteur adénoviral selon l'une quelconque des revendications 1 à 10, pour la production d'un vaccin.


### SEQUENCE LISTING

<110> Københavns Universitet
<120> Increase of immune response from adenoviral vectors
<130> P1124DK00
<160> 10
<170> PatentIn version 3.1
<210> 1
   <211> 1287
   <212> DNA
   <213> Homo sapiens
<220>
   <221> CDS
   <222> (100)..(750)
   <223>
<400> 1
   ctgcaggggg gggggggggg gggggggaca ttggctcttc cttggggagt gatgcacagg      60
   aggagaagca ggagctgtcg ggaagatcag aagccagtc atg gat gac cag cgc        114
                                              Met Asp Asp Gln Arg
                                              1               5
   gac ctt atc tcc aac aat gag caa ctg ccc atg ctg ggc cgg cgc cct      162
   Asp Leu Ile Ser Asn Asn Glu Gln Leu Pro Met Leu Gly Arg Arg Pro
                   10                  15                  20
   ggg gcc ccg gag agc aag tgc agc cgc gga gcc ctg tac aca ggc ttt      210
   Gly Ala Pro Glu Ser Lys Cys Ser Arg Gly Ala Leu Tyr Thr Gly Phe
               25                  30                  35
   tcc atc ctg gtg act ctg ctc ctc gct ggc cag gcc acc acc gcc tac      258
   Ser Ile Leu Val Thr Leu Leu Leu Ala Gly Gln Ala Thr Thr Ala Tyr
           40                  45                  50
   ttc ctg tac cag cag cag ggc cgg ctg gac aaa ctg aca gtc acc tcc      306
   Phe Leu Tyr Gln Gln Gln Gly Arg Leu Asp Lys Leu Thr Val Thr Ser
       55                  60                  65
   cag aac ctg cag ctg gag aac ctg cgc atg aag ctt ccc aag cct ccc       354
   Gln Asn Leu Gln Leu Glu Asn Leu Arg Met Lys Leu Pro Lys Pro Pro
   70                  75                  80                  85
   aag cct gtg agc aag atg cgc atg gcc acc ccg ctg ctg atg cag gcg       402
   Lys Pro Val Ser Lys Met Arg Met Ala Thr Pro Leu Leu Met Gln Ala
                   90                  95                  100
   ctg ccc atg gga gcc ctg ccc cag ggg ccc atg cag aat gcc acc aag       450
   Leu Pro Met Gly Ala Leu Pro Gln Gly Pro Met Gln Asn Ala Thr Lys
               105                 110                 115
   tat ggc aac atg aca gag gac cat gtg atg cac ctg ctc cag aat gct       498
   Tyr Gly Asn Met Thr Glu Asp His Val Met His Leu Leu Gln Asn Ala
           120                 125                 130
   gac ccc ctg aag gtg tac ccg cca ctg aag ggg agc ttc ccg gag aac       546
   Asp Pro Leu Lys Val Tyr Pro Pro Leu Lys Gly Ser Phe Pro Glu Asn
       135                 140                 145
   ctg aga cac ctt aag aac acc atg gag acc ata gac tgg aag gtc ttt       594
   Leu Arg His Leu Lys Asn Thr Met Glu Thr Ile Asp Trp Lys Val Phe
   150                 155                 160                 165
   gag agc tgg atg cac cat tgg ctc ctg ttt gaa atg agc agg cac tcc       642
   Glu Ser Trp Met His His Trp Leu Leu Phe Glu Met Ser Arg His Ser
                   170                 175                 180
   ttg gag caa aag ccc act gac gct cca ccg aaa gag tca ctg gaa ctg       690
   Leu Glu Gln Lys Pro Thr Asp Ala Pro Pro Lys Glu Ser Leu Glu Leu
               185                 190                 195
   gag gac ccg tct tct ggg ctg ggt gtg acc aag cag gat ctg ggc cca       738
   Glu Asp Pro Ser Ser Gly Leu Gly Val Thr Lys Gln Asp Leu Gly Pro
           200                 205                 210
   gtc ccc atg tga gagcagcaga ggcggtcttc aacatcctgc cagccccaca           790
   Val Pro Met
       215
   cagctacagc tttcttgctc ccttcagccc ccagcccctc ccccatctcc caccctgtac     850
   ctcatcccat gagaccctgg tgcctggctc tttcgtcacc cttggacaag acaaaccaag     910
   tcggaacagc agataacaat gcagcaaggc cctgctgccc aatctccatc tgtcaacagg     970
   ggcgtgaggt cccaggaagt ggccaaaagc tagacagatc cccgttcctg acatcacagc    1030
   agcctccaac acaaggctcc aagacctagg ctcatggacg agatgggaag gcacagggag    1090
   aagggataac cctacaccca gaccccaggc tggacatgct gactgtcctc tcccctccag    1150
   cctttggcct tggcttttct agcctattta cctgcaggct gagccactct cttccctttc    1210
   cccagcatca ctccccaagg aagagccaat gttttccacc catccctccc cccccccccc    1270
   cccccccccc cctgcag                                                   1287
<210> 2
   <211> 216
   <212> PRT
   <213> Homo sapiens
<400> 2
   Met Asp Asp Gln Arg Asp Leu Ile Ser Asn Asn Glu Gln Leu Pro Met
   1               5                   10                  15
   Leu Gly Arg Arg Pro Gly Ala Pro Glu Ser Lys Cys Ser Arg Gly Ala
               20                  25                  30
   Leu Tyr Thr Gly Phe Ser Ile Leu Val Thr Leu Leu Leu Ala Gly Gln
           35                  40                  45
   Ala Thr Thr Ala Tyr Phe Leu Tyr Gln Gln Gln Gly Arg Leu Asp Lys
       50                  55                  60
   Leu Thr Val Thr Ser Gln Asn Leu Gln Leu Glu Asn Leu Arg Met Lys
   65                  70                  75                  80
   Leu Pro Lys Pro Pro Lys Pro Val Ser Lys Met Arg Met Ala Thr Pro
                   85                  90                  95
   Leu Leu Met Gln Ala Leu Pro Met Gly Ala Leu Pro Gln Gly Pro Met
               100                 105                 110
   Gln Asn Ala Thr Lys Tyr Gly Asn Met Thr Glu Asp His Val Met His
           115                 120                 125
   Leu Leu Gln Asn Ala Asp Pro Leu Lys Val Tyr Pro Pro Leu Lys Gly
       130                 135                 140
   Ser Phe Pro Glu Asn Leu Arg His Leu Lys Asn Thr Met Glu Thr Ile
   145                 150                 155                 160
   Asp Trp Lys Val Phe Glu Ser Trp Met His His Trp Leu Leu Phe Glu
                   165                 170                 175
   Met Ser Arg His Ser Leu Glu Gln Lys Pro Thr Asp Ala Pro Pro Lys
               180                 185                 190
   Glu Ser Leu Glu Leu Glu Asp Pro Ser Ser Gly Leu Gly Val Thr Lys
           195                 200                 205
   Gln Asp Leu Gly Pro Val Pro Met
       210                 215
<210> 3
   <211> 648
   <212> DNA
   <213> Mus musculus
<220>
   <221> CDS
   <222> (1)..(648)
   <223>
<400> 3
   atg gat gac caa cgc gac ctc atc tct aac cat gaa cag ttg ccc ata      48
   Met Asp Asp Gln Arg Asp Leu Ile Ser Asn His Glu Gln Leu Pro Ile
   1               5                   10                  15
   ctg ggc aac cgc cct aga gag cca gaa agg tgc agc cgt gga gct ctg      96
   Leu Gly Asn Arg Pro Arg Glu Pro Glu Arg Cys Ser Arg Gly Ala Leu
               20                  25                  30
   tac acc ggt gtc tct gtc ctg gtg gct ctg ctc ttg gct ggg cag gcc      144
   Tyr Thr Gly Val Ser Val Leu Val Ala Leu Leu Leu Ala Gly Gln Ala
           35                  40                  45
   acc act gct tac ttc ctg tac cag caa cag ggc cgc cta gac aag ctg      192
   Thr Thr Ala Tyr Phe Leu Tyr Gln Gln Gln Gly Arg Leu Asp Lys Leu
       50                  55                  60
   acc atc acc tcc cag aac ctg caa ctg gag agc ctt cgc atg aag ctt      240
   Thr Ile Thr Ser Gln Asn Leu Gln Leu Glu Ser Leu Arg Met Lys Leu
   65                  70                  75                  80
   ccg aaa tct gcc aaa cct gtg agc cag atg cgg atg gct act ccc ttg      288
   Pro Lys Ser Ala Lys Pro Val Ser Gln Met Arg Met Ala Thr Pro Leu
                   85                  90                  95
   ctg atg cgt cca atg tcc atg gat aac atg ctc ctt ggg cct gtg aag      336
   Leu Met Arg Pro Met Ser Met Asp Asn Met Leu Leu Gly Pro Val Lys
               100                 105                 110
   aac gtt acc aag tac ggc aac atg acc cag gac cat gtg atg cat ctg      384
   Asn Val Thr Lys Tyr Gly Asn Met Thr Gln Asp His Val Met His Leu
           115                 120                 125
   ctc acg agg tct gga ccc ctg gag tac ccg cag ctg aag ggg acc ttc      432
   Leu Thr Arg Ser Gly Pro Leu Glu Tyr Pro Gln Leu Lys Gly Thr Phe
       130                 135                 140
   cca gag aat ctg aag cat ctt aag aac tcc atg gat ggc gtg aac tgg      480
   Pro Glu Asn Leu Lys His Leu Lys Asn Ser Met Asp Gly Val Asn Trp
   145                 150                 155                 160
   aag atc ttc gag agc tgg atg aag cag tgg ctc ttg ttt gag atg agc      528
   Lys Ile Phe Glu Ser Trp Met Lys Gln Trp Leu Leu Phe Glu Met Ser
                   165                 170                 175
   aag aac tcc ctg gag gag aag aag ccc acc gag gct cca cct aaa gag      576
   Lys Asn Ser Leu Glu Glu Lys Lys Pro Thr Glu Ala Pro Pro Lys Glu
               180                 185                 190
   cca ctg gac atg gaa gac cta tct tct ggc ctg gga gtg acc agg cag      624
   Pro Leu Asp Met Glu Asp Leu Ser Ser Gly Leu Gly Val Thr Arg Gln
           195                 200                 205
   gaa ctg ggt caa gtc acc ctg tga                                      648
   Glu Leu Gly Gln Val Thr Leu
       210                 215
<210> 4
   <211> 215
   <212> PRT
   <213> Mus musculus
<400> 4
   Met Asp Asp Gln Arg Asp Leu Ile Ser Asn His Glu Gln Leu Pro Ile
   1               5                   10                  15
   Leu Gly Asn Arg Pro Arg Glu Pro Glu Arg Cys Ser Arg Gly Ala Leu
               20                  25                  30
   Tyr Thr Gly Val Ser Val Leu Val Ala Leu Leu Leu Ala Gly Gln Ala
           35                  40                  45
   Thr Thr Ala Tyr Phe Leu Tyr Gln Gln Gln Gly Arg Leu Asp Lys Leu
       50                  55                  60
   Thr Ile Thr Ser Gln Asn Leu Gln Leu Glu Ser Leu Arg Met Lys Leu
   65                  70                  75                  80
   Pro Lys Ser Ala Lys Pro Val Ser Gln Met Arg Met Ala Thr Pro Leu
                   85                  90                  95
   Leu Met Arg Pro Met Ser Met Asp Asn Met Leu Leu Gly Pro Val Lys
               100                 105                 110
   Asn Val Thr Lys Tyr Gly Asn Met Thr Gln Asp His Val Met His Leu
           115                 120                 125
   Leu Thr Arg Ser Gly Pro Leu Glu Tyr Pro Gln Leu Lys Gly Thr Phe
       130                 135                 140
   Pro Glu Asn Leu Lys His Leu Lys Asn Ser Met Asp Gly Val Asn Trp
   145                 150                 155                 160
   Lys Ile Phe Glu Ser Trp Met Lys Gln Trp Leu Leu Phe Glu Met Ser
                   165                 170                 175
   Lys Asn Ser Leu Glu Glu Lys Lys Pro Thr Glu Ala Pro Pro Lys Glu
               180                 185                 190
   Pro Leu Asp Met Glu Asp Leu Ser Ser Gly Leu Gly Val Thr Arg Gln
           195                 200                 205
   Glu Leu Gly Gln Val Thr Leu
       210                 215
<210> 5
   <211> 2587
   <212> DNA
   <213> Artificial Sequence
<220>
   <223> Vector number 574
<400> 5
   tgtatcatat gccaagtccg ccccctattg acgtcaatga cggtaaatgg cccgcctggc     60
   attatgccca gtacatgacc ttacgggact ttcctacttg gcagtacatc tacgtattag    120
   tcatcgctat taccatggtg atgcggtttt ggcagtacac caatgggcgt ggatagcggt    180
   ttgactcacg gggatttcca agtctccacc ccattgacgt caatgggagt ttgttttggc    240
   accaaaatca acgggacttt ccaaaatgtc gtaataaccc cgccccgttg acgcaaatgg    300
   gcggtaggcg tgtacggtgg gaggtctata taagcagagc tcgtttagtg aaccgtcaga    360
   tccggtcgcg cgaattcatg ggtcagattg tgacaatgtt tgaggctctg cctcacatca    420
   tcgatgaggt gatcaacatt gtcattattg tgcttatcgt gatcacgggt atcaaggctg    480
   tctacaattt tgccacctgt gggatattcg cattgatcag tttcctactt ctggctggca    540
   ggtcctgtgg catgtacggt cttaagggac ccgacattta caaaggagtt taccaattta    600
   agtcagtgga gtttgatatg tcacatctga acctgaccat gcccaacgca tgttcagcca    660
   acaactccca ccattacatc agtatgggga cttctggact agaattgacc ttcaccaatg    720
   attccatcat cagtcacaac ttttgcaatc tgacctctgc cttcaacaaa aagacctttg    780
   accacacact catgagtata gtttcgagcc tacacctcag tatcagaggg aactccaact    840
   ataaggcagt atcctgcgac ttcaacaatg gcataaccat ccaatacaac ttgacattct    900
   cagatcgaca aagtgctcag agccagtgta gaaccttcag aggtagagtc ctagatatgt    960
   ttagaactgc cttcgggggg aaatacatga ggagtggctg gggctggaca ggctcagatg   1020
   gcaagaccac ctggtgtagc cagacgagtt accaatacct gattatacaa aatagaacct    1080
   gggaaaacca ctgcacatat gcaggtcctt ttgggatgtc caggattctc ctttcccaag    1140
   agaagactaa gttcttcact aggagactag cgggcacatt cacctggact ttgtcagact    1200
   cttcaggggt ggagaatcca ggtggttatt gcctgaccaa atggatgatt cttgctgcag    1260
   agcttaagtg tttcgggaac acagcagttg cgaaatgcaa tgtaaatcat gatgccgaat    1320
   tctgtgacat gctgcgacta attgactaca acaaggctgc tttgagtaag ttcaaagagg    1380
   acgtagaatc tgccttgcac ttattcaaaa caacagtgaa ttctttgatt tcagatcaac    1440
   tactgatgag gaaccacttg agagatctga tgggggtgcc atattgcaat tactcaaagt    1500
   tttggtacct agaacatgca aagaccggcg aaactagtgt ccccaagtgc tggcttgtca    1560
   ccaatggttc ttacttaaat gagacccact tcagtgatca aatcgaacag gaagccgata    1620
   acatgattac agagatgttg aggaaggatt acataaagag gcaggggagt acccccctag    1680
   cattgatgga ccttctgatg ttttccacat ctgcatatct agtcagcatc ttcctgcacc    1740
   ttgtcaaaat accaacacac aggcacataa aaggtggctc atgtccaaag ccacaccgat    1800
   taaccaacaa aggaatttgt agttgtggtg catttaaggt gcctggtgta aaaaccgtct    1860
   ggaaaagacg ctgaggtacc cggggatcct ctagagtcga cctgcaggca tgcaagcttg    1920
   ggatctttgt gaaggaacct tacttctgtg gtgtgacata attggacaaa ctacctacag    1980
   agatttaaag ctctaaggta aatataaaat ttttaagtgt ataatgtgtt aaactactga    2040
   ttctaattgt ttgtgtattt tagattcaca gtcccaaggc tcatttcagg cccctcagtc    2100
   ctcacagtct gttcatgatc ataatcagcc ataccacatt tgtagaggtt ttacttgctt    2160
   taaaaaacct cccacacctc cccctgaacc tgaaacataa aatgaatgca attgttgttg    2220
   ttaacttgtt tattgcagct tataatggtt acaaataaag caatagcatc acaaatttca    2280
   caaataaagc atttttttca ctgcattcta gttgtggttt gtccaaactc atcaatgtat    2340
   cttatcatgt ctggatcgcg gccgcctaga gggaaggtgc tgaggtacga tgagacccgc    2400
   accaggtgca gaccctgcga gtgtggcggt aaacatatta ggaaccagcc tgtgatgctg    2460
   gatgtgaccg aggagctgag gcccgatcac ttggtgctgg cctgcacccg cgctgagttt    2520
   ggctctagcg atgaagatac agattgaggt actgaaatgt gtgggcgtgg cttaagggtg    2580
   ggaaaga                                                              2587
<210> 6
   <211> 3232
   <212> DNA
   <213> Artificial Sequence
<220>
   <223> Vector number 578
<400> 6
   tgtatcatat gccaagtccg ccccctattg acgtcaatga cggtaaatgg cccgcctggc      60
   attatgccca gtacatgacc ttacgggact ttcctacttg gcagtacatc tacgtattag     120
   tcatcgctat taccatggtg atgcggtttt ggcagtacac caatgggcgt ggatagcggt     180
   ttgactcacg gggatttcca agtctccacc ccattgacgt caatgggagt ttgttttggc     240
   accaaaatca acgggacttt ccaaaatgtc gtaataaccc cgccccgttg acgcaaatgg     300
   gcggtaggcg tgtacggtgg gaggtctata taagcagagc tcgtttagtg aaccgtcaga     360
   tccggtcgcg cgaattcatg gatgaccaac gcgacctcat ctctaaccat gaacagttgc     420
   ccatactggg caaccgccct agagagccag aaaggtgcag ccgtggagct ctgtacaccg     480
   gtgtctctgt cctggtggct ctgctcttgg ctgggcaggc caccactgct tacttcctgt     540
   accagcaaca gggccgccta gacaagctga ccatcacctc ccagaacctg caactggaga     600
   gccttcgcat gaagcttccg aaatctgcca aacctgtgag ccagatgcgg atggctactc     660
   ccttgctgat gcgtccaatg tccatggata acatgctcct tgggcctgtg aagaacgtta     720
   ccaagtacgg caacatgacc caggaccatg tgatgcatct gctcacgagg tctggacccc     780
   tggagtaccc gcagctgaag gggaccttcc cagagaatct gaagcatctt aagaactcca     840
   tggatggcgt gaactggaag atcttcgaga gctggatgaa gcagtggctc ttgtttgaga     900
   tgagcaagaa ctccctggag gagaagaagc ccaccgaggc tccacctaaa gagccactgg     960
   acatggaaga cctatcttct ggcctgggag tgaccaggca ggaactgggt caagtcaccc    1020
   tgatgggtca gattgtgaca atgtttgagg ctctgcctca catcatcgat gaggtgatca    1080
   acattgtcat tattgtgctt atcgtgatca cgggtatcaa ggctgtctac aattttgcca    1140
   cctgtgggat attcgcattg atcagtttcc tacttctggc tggcaggtcc tgtggcatgt    1200
   acggtcttaa gggacccgac atttacaaag gagtttacca atttaagtca gtggagtttg    1260
   atatgtcaca tctgaacctg accatgccca acgcatgttc agccaacaac tcccaccatt    1320
   acatcagtat ggggacttct ggactagaat tgaccttcac caatgattcc atcatcagtc    1380
   acaacttttg caatctgacc tctgccttca acaaaaagac ctttgaccac acactcatga    1440
   gtatagtttc gagcctacac ctcagtatca gagggaactc caactataag gcagtatcct    1500
   gcgacttcaa caatggcata accatccaat acaacttgac attctcagat cgacaaagtg    1560
   ctcagagcca gtgtagaacc ttcagaggta gagtcctaga tatgtttaga actgccttcg    1620
   gggggaaata catgaggagt ggctggggct ggacaggctc agatggcaag accacctggt    1680
   gtagccagac gagttaccaa tacctgatta tacaaaatag aacctgggaa aaccactgca    1740
   catatgcagg tccttttggg atgtccagga ttctcctttc ccaagagaag actaagttct    1800
   tcactaggag actagcgggc acattcacct ggactttgtc agactcttca ggggtggaga   1860
   atccaggtgg ttattgcctg accaaatgga tgattcttgc tgcagagctt aagtgtttcg   1920
   ggaacacagc agttgcgaaa tgcaatgtaa atcatgatgc cgaattctgt gacatgctgc   1980
   gactaattga ctacaacaag gctgctttga gtaagttcaa agaggacgta gaatctgcct   2040
   tgcacttatt caaaacaaca gtgaattctt tgatttcaga tcaactactg atgaggaacc   2100
   acttgagaga tctgatgggg gtgccatatt gcaattactc aaagttttgg tacctagaac   2160
   atgcaaagac cggcgaaact agtgtcccca agtgctggct tgtcaccaat ggttcttact   2220
   taaatgagac ccacttcagt gatcaaatcg aacaggaagc cgataacatg attacagaga   2280
   tgttgaggaa ggattacata aagaggcagg ggagtacccc cctagcattg atggaccttc   2340
   tgatgttttc cacatctgca tatctagtca gcatcttcct gcaccttgtc aaaataccaa   2400
   cacacaggca cataaaaggt ggctcatgtc caaagccaca ccgattaacc aacaaaggaa   2460
   tttgtagttg tggtgcattt aaggtgcctg gtgtaaaaac cgtctggaaa agacgctgag   2520
   gtacccgggg atcctctaga gtcgacctgc aggcatgcaa gcttgggatc tttgtgaagg   2580
   aaccttactt ctgtggtgtg acataattgg acaaactacc tacagagatt taaagctcta   2640
   aggtaaatat aaaattttta agtgtataat gtgttaaact actgattcta attgtttgtg   2700
   tattttagat tcacagtccc aaggctcatt tcaggcccct cagtcctcac agtctgttca   2760
   tgatcataat cagccatacc acatttgtag aggttttact tgctttaaaa aacctcccac   2820
   acctccccct gaacctgaaa cataaaatga atgcaattgt tgttgttaac ttgtttattg   2880
   cagcttataa tggttacaaa taaagcaata gcatcacaaa tttcacaaat aaagcatttt   2940
   tttcactgca ttctagttgt ggtttgtcca aactcatcaa tgtatcttat catgtctgga   3000
   tcgcggccgc ctagagggaa ggtgctgagg tacgatgaga cccgcaccag gtgcagaccc   3060
   tgcgagtgtg gcggtaaaca tattaggaac cagcctgtga tgctggatgt gaccgaggag   3120
   ctgaggcccg atcacttggt gctggcctgc acccgcgctg agtttggctc tagcgatgaa   3180
   gatacagatt gaggtactga aatgtgtggg cgtggcttaa gggtgggaaa ga           3232
<210> 7
   <211> 1145
   <212> DNA
   <213> Artificial Sequence
<220>
   <223> Vector number 768
<400> 7
   tgtatcatat gccaagtccg ccccctattg acgtcaatga cggtaaatgg cccgcctggc     60
   attatgccca gtacatgacc ttacgggact ttcctacttg gcagtacatc tacgtattag     120
   tcatcgctat taccatggtg atgcggtttt ggcagtacac caatgggcgt ggatagcggt     180
   ttgactcacg gggatttcca agtctccacc ccattgacgt caatgggagt ttgttttggc     240
   accaaaatca acgggacttt ccaaaatgtc gtaataaccc cgccccgttg acgcaaatgg     300
   gcggtaggcg tgtacggtgg gaggtctata taagcagagc tcgtttagtg aaccgtcaga     360
   tccggtcgcg cgaattgtta attaagcgat cgctatttaa ataggcgcgc cagtttaaac     420
   aggccggcct aaggtacccg gggatcctct agagtcgacc tgcaggcatg caagcttggg     480
   atctttgtga aggaacctta cttctgtggt gtgacataat tggacaaact acctacagag     540
   atttaaagct ctaaggtaaa tataaaattt ttaagtgtat aatgtgttaa actactgatt     600
   ctaattgttt gtgtatttta gattcacagt cccaaggctc atttcaggcc cctcagtcct     660
   cacagtctgt tcatgatcat aatcagccat accacatttg tagaggtttt acttgcttta     720
   aaaaacctcc cacacctccc cctgaacctg aaacataaaa tgaatgcaat tgttgttgtt     780
   aacttgttta ttgcagctta taatggttac aaataaagca atagcatcac aaatttcaca     840
   aataaagcat ttttttcact gcattctagt tgtggtttgt ccaaactcat caatgtatct     900
   tatcatgtct ggatcgcggc cgcctagagg gaaggtgctg aggtacgatg agacccgcac     960
   caggtgcaga ccctgcgagt gtggcggtaa acatattagg aaccagcctg tgatgctgga    1020
   tgtgaccgag gagctgaggc ccgatcactt ggtgctggcc tgcacccgcg ctgagtttgg    1080
   ctctagcgat gaagatacag attgaggtac tgaaatgtgt gggcgtggct taagggtggg    1140
   aaaga                                                                 1145
<210> 8
   <211> 1783
   <212> DNA
   <213> Artificial Sequence
<220>
   <223> Vector number 770
<400> 8
   tgtatcatat gccaagtccg ccccctattg acgtcaatga cggtaaatgg cccgcctggc     60
   attatgccca gtacatgacc ttacgggact ttcctacttg gcagtacatc tacgtattag     120
   tcatcgctat taccatggtg atgcggtttt ggcagtacac caatgggcgt ggatagcggt     180
   ttgactcacg gggatttcca agtctccacc ccattgacgt caatgggagt ttgttttggc     240
   accaaaatca acgggacttt ccaaaatgtc gtaataaccc cgccccgttg acgcaaatgg     300
   gcggtaggcg tgtacggtgg gaggtctata taagcagagc tcgtttagtg aaccgtcaga    360
   tccggtcgcg cgaattcatg gatgaccaac gcgacctcat ctctaaccat gaacagttgc    420
   ccatactggg caaccgccct agagagccag aaaggtgcag ccgtggagct ctgtacaccg    480
   gtgtctctgt cctggtggct ctgctcttgg ctgggcaggc caccactgct tacttcctgt    540
   accagcaaca gggccgccta gacaagctga ccatcacctc ccagaacctg caactggaga    600
   gccttcgcat gaagcttccg aaatctgcca aacctgtgag ccagatgcgg atggctactc    660
   ccttgctgat gcgtccaatg tccatggata acatgctcct tgggcctgtg aagaacgtta    720
   ccaagtacgg caacatgacc caggaccatg tgatgcatct gctcacgagg tctggacccc    780
   tggagtaccc gcagctgaag gggaccttcc cagagaatct gaagcatctt aagaactcca     840
   tggatggcgt gaactggaag atcttcgaga gctggatgaa gcagtggctc ttgtttgaga    900
   tgagcaagaa ctccctggag gagaagaagc ccaccgaggc tccacctaaa gagccactgg    960
   acatggaaga cctatcttct ggcctgggag tgaccaggca ggaactgggt caagtcaccc   1020
   tgagcgatcg ctatttaaat aggcgcgcca gtttaaacag gccggcctaa ggtacccggg   1080
   gatcctctag agtcgacctg caggcatgca agcttgggat ctttgtgaag gaaccttact   1140
   tctgtggtgt gacataattg gacaaactac ctacagagat ttaaagctct aaggtaaata   1200
   taaaattttt aagtgtataa tgtgttaaac tactgattct aattgtttgt gtattttaga   1260
   ttcacagtcc caaggctcat ttcaggcccc tcagtcctca cagtctgttc atgatcataa   1320
   tcagccatac cacatttgta gaggttttac ttgctttaaa aaacctccca cacctccccc   1380
   tgaacctgaa acataaaatg aatgcaattg ttgttgttaa cttgtttatt gcagcttata   1440
   atggttacaa ataaagcaat agcatcacaa atttcacaaa taaagcattt ttttcactgc   1500
   attctagttg tggtttgtcc aaactcatca atgtatctta tcatgtctgg atcgcggccg   1560
   cctagaggga aggtgctgag gtacgatgag acccgcacca ggtgcagacc ctgcgagtgt   1620
   ggcggtaaac atattaggaa ccagcctgtg atgctggatg tgaccgagga gctgaggccc   1680
   gatcacttgg tgctggcctg cacccgcgct gagtttggct ctagcgatga agatacagat   1740
   tgaggtactg aaatgtgtgg gcgtggctta agggtgggaa aga                     1783
<210> 9
   <211> 2397
   <212> DNA
   <213> Artificial Sequence
<220>
   <223> Vector termed pacCMV Ii MCS IRES2
<400> 9
   tgtatcatat gccaagtccg ccccctattg acgtcaatga cggtaaatgg cccgcctggc     60
   attatgccca gtacatgacc ttacgggact ttcctacttg gcagtacatc tacgtattag    120
   tcatcgctat taccatggtg atgcggtttt ggcagtacac caatgggcgt ggatagcggt    180
   ttgactcacg gggatttcca agtctccacc ccattgacgt caatgggagt ttgttttggc    240
   accaaaatca acgggacttt ccaaaatgtc gtaataaccc cgccccgttg acgcaaatgg    300
   gcggtaggcg tgtacggtgg gaggtctata taagcagagc tcgtttagtg aaccgtcaga    360
   tccggtcgcg cgaattcatg gatgaccaac gcgacctcat ctctaaccat gaacagttgc    420
   ccatactggg caaccgccct agagagccag aaaggtgcag ccgtggagct ctgtacaccg    480
   gtgtctctgt cctggtggct ctgctcttgg ctgggcaggc caccactgct tacttcctgt    540
   accagcaaca gggccgccta gacaagctga ccatcacctc ccagaacctg caactggaga    600
   gccttcgcat gaagcttccg aaatctgcca aacctgtgag ccagatgcgg atggctactc    660
   ccttgctgat gcgtccaatg tccatggata acatgctcct tgggcctgtg aagaacgtta    720
   ccaagtacgg caacatgacc caggaccatg tgatgcatct gctcacgagg tctggacccc    780
   tggagtaccc gcagctgaag gggaccttcc cagagaatct gaagcatctt aagaactcca    840
   tggatggcgt gaactggaag atcttcgaga gctggatgaa gcagtggctc ttgtttgaga    900
   tgagcaagaa ctccctggag gagaagaagc ccaccgaggc tccacctaaa gagccactgg    960
   acatggaaga cctatcttct ggcctgggag tgaccaggca ggaactgggt caagtcaccc   1020
   tgagcgatcg ctatttaaat aggcgcgcca gtttaaacag gccggcctaa ggtacccggg   1080
   gatccgcccc tctccctccc ccccccctaa cgttactggc cgaagccgct tggaataagg   1140
   ccggtgtgcg tttgtctata tgttattttc caccatattg ccgtcttttg gcaatgtgag   1200
   ggcccggaaa cctggccctg tcttcttgac gagcattcct aggggtcttt cccctctcgc   1260
   caaaggaatg caaggtctgt tgaatgtcgt gaaggaagca gttcctctgg aagcttcttg   1320
   aagacaaaca acgtctgtag cgaccctttg caggcagcgg aaccccccac ctggcgacag   1380
   gtgcctctgc ggccaaaagc cacgtgtata agatacacct gcaaaggcgg cacaacccca   1440
   gtgccacgtt gtgagttgga tagttgtgga aagagtcaaa tggctctcct caagcgtatt   1500
   caacaagggg ctgaaggatg cccagaaggt accccattgt atgggatctg atctggggcc   1560
   tcggtgcaca tgctttacat gtgtttagtc gaggttaaaa aaacgtctag gccccccgaa   1620
   ccacggggac gtggttttcc tttgaaaaac acgatgataa tatggccaca acctagggat   1680
   aacagggtaa tgcccgggct ctagagtcga cctgcaggca tgcaagcttg ggatctttgt   1740
   gaaggaacct tacttctgtg gtgtgacata attggacaaa ctacctacag agatttaaag   1800
   ctctaaggta aatataaaat ttttaagtgt ataatgtgtt aaactactga ttctaattgt   1860
   ttgtgtattt tagattcaca gtcccaaggc tcatttcagg cccctcagtc ctcacagtct   1920
   gttcatgatc ataatcagcc ataccacatt tgtagaggtt ttacttgctt taaaaaacct 1980
   cccacacctc cccctgaacc tgaaacataa aatgaatgca attgttgttg ttaacttgtt 2040
   tattgcagct tataatggtt acaaataaag caatagcatc acaaatttca caaataaagc 2100
   atttttttca ctgcattcta gttgtggttt gtccaaactc atcaatgtat cttatcatgt 2160
   ctggatcgcg gccgcctaga gggaaggtgc tgaggtacga tgagacccgc accaggtgca 2220
   gaccctgcga gtgtggcggt aaacatatta ggaaccagcc tgtgatgctg gatgtgaccg 2280
   aggagctgag gcccgatcac ttggtgctgg cctgcacccg cgctgagttt ggctctagcg 2340
   atgaagatac agattgaggt actgaaatgt gtgggcgtgg cttaagggtg ggaaaga 2397
<210> 10
   <211> 1752
   <212> DNA
   <213> Artificial Sequence
<220>
   <223> Vector termed pacCMV MCS IRES2
<400> 10
   tgtatcatat gccaagtccg ccccctattg acgtcaatga cggtaaatgg cccgcctggc 60
   attatgccca gtacatgacc ttacgggact ttcctacttg gcagtacatc tacgtattag 120
   tcatcgctat taccatggtg atgcggtttt ggcagtacac caatgggcgt ggatagcggt 180
   ttgactcacg gggatttcca agtctccacc ccattgacgt caatgggagt ttgttttggc 240
   accaaaatca acgggacttt ccaaaatgtc gtaataaccc cgccccgttg acgcaaatgg 300
   gcggtaggcg tgtacggtgg gaggtctata taagcagagc tcgtttagtg aaccgtcaga 360
   tccggtcgcg cgaattcagc gatcgctatt taaataggcg cgccagttta aacaggccgg 420
   cctaaggtac ccggggatcc gcccctctcc ctcccccccc cctaacgtta ctggccgaag 480
   ccgcttggaa taaggccggt gtgcgtttgt ctatatgtta ttttccacca tattgccgtc 540
   ttttggcaat gtgagggccc ggaaacctgg ccctgtcttc ttgacgagca ttcctagggg 600
   tctttcccct ctcgccaaag gaatgcaagg tctgttgaat gtcgtgaagg aagcagttcc 660
   tctggaagct tcttgaagac aaacaacgtc tgtagcgacc ctttgcaggc agcggaaccc 720
   cccacctggc gacaggtgcc tctgcggcca aaagccacgt gtataagata cacctgcaaa 780
   ggcggcacaa ccccagtgcc acgttgtgag ttggatagtt gtggaaagag tcaaatggct 840
   ctcctcaagc gtattcaaca aggggctgaa ggatgcccag aaggtacccc attgtatggg 900
   atctgatctg gggcctcggt gcacatgctt tacatgtgtt tagtcgaggt taaaaaaacg 960
   tctaggcccc ccgaaccacg gggacgtggt tttcctttga aaacacgatg gataatatgg 1020
   ccacaaccta gggataacag ggtaatgccc gggctctaga gtcgacctgc aggcatgcaa   1080
   gcttgggatc tttgtgaagg aaccttactt ctgtggtgtg acataattgg acaaactacc   1140
   tacagagatt taaagctcta aggtaaatat aaaattttta agtgtataat gtgttaaact   1200
   actgattcta attgtttgtg tattttagat tcacagtccc aaggctcatt tcaggcccct   1260
   cagtcctcac agtctgttca tgatcataat cagccatacc acatttgtag aggttttact   1320
   tgctttaaaa aacctcccac acctccccct gaacctgaaa cataaaatga atgcaattgt   1380
   tgttgttaac ttgtttattg cagcttataa tggttacaaa taaagcaata gcatcacaaa   1440
   tttcacaaat aaagcatttt tttcactgca ttctagttgt ggtttgtcca aactcatcaa   1500
   tgtatcttat catgtctgga tcgcggccgc ctagagggaa ggtgctgagg tacgatgaga   1560
   cccgcaccag gtgcagaccc tgcgagtgtg gcggtaaaca tattaggaac cagcctgtga   1620
   tgctggatgt gaccgaggag ctgaggcccg atcacttggt gctggcctgc acccgcgctg   1680
   agtttggctc tagcgatgaa gatacagatt gaggtactga aatgtgtggg cgtggcttaa   1740
   gggtgggaaa ga                                                       1752